# EUROPEAN PATENT APPLICATION

(11) **EP 4 195 138 A1**
(43) Date of publication of application: **14.06.2023**
(21) Application number: 20948137.3
(22) Date of filing: 05.08.2020
(51) Int. Cl.: G06Q 50/10, A61B 5/16, G16H 15/00, G16H 20/70

(54) **INFORMATION PROCESSING DEVICE, INFORMATION PROCESSING METHOD, AND PROGRAM**

(71) Applicant: Japan Tobacco Inc., Tokyo 105-6927 (JP)
(72) Inventor: FUJINO, Yusuke, Tokyo 130-8603 (JP); HARA, Kenta, Tokyo 130-8603 (JP); SHIMADA, Yurina, Tokyo 130-8603 (JP); KATO, Masato, Tokyo 130-8603 (JP)
(74) Representative: Hoffmann Eitle
(86) International application number: PCT/JP2020/030043
(87) International publication number: WO 2022/029935

(57) **Abstract**

This information processing device comprises: an acquisition unit that acquires at least one index value based on biological data measured by a scent/flavor inhaler; a comparison unit that compares the at least one index value to a threshold value; a creation unit that creates, on the basis of the comparison result of the comparison unit, stress state information based on the at least one index value; a selection unit that selects at least one recommendation in accordance with the at least one index value; and a display control unit that controls the display of the stress state information created by the creation unit and the at least one recommendation selected by the selection unit.

## Description

### FIELD

The present invention relates to an information processing device, an information processing method, and a program.

### BACKGROUND

In recent years, a technology for collecting health data by mounting a vital sensor on a device has been developed.

For example, Patent Literature 1 discloses that a vital sensor capable of measuring a heart rate or the like is mounted on a device or a case thereof, the heart rate or the like of a user is collected, and such collected health data is linked to a stress index.

Patent Literature 2 discloses that an electronic cigarette receives a state of a user in real time by a biosensor and controls an amount of substance, a substance characteristic, and a period of use of the electronic cigarette.

### CITATION LIST

### PATENT LITERATURE

[Patent Literature 1] International Publication No. 2019/175810
[Patent Literature 2] International Publication No. 2018/098371

### SUMMARY

### TECHNICAL PROBLEM

However, in Patent Literature 1, although the collected health data is associated with the stress index, the user of the device cannot take an appropriate action for stress relief according to the stress index.

In addition, in Patent Literature 2, an output for controlling the substance amount, the substance characteristic, and the period of use is performed according to the state of the user, but information for performing an appropriate action for stress relief according to a stress index is not provided.

In view of the above problem, an object of the present invention is to provide a technique that can recommend a user of a device capable of collecting predetermined data to perform an appropriate action for stress relief.

### SOLUTION TO PROBLEM

An information processing device according to an aspect of the present invention includes: an acquisition unit configured to acquire at least one index value based on biometric data measured by a flavor inhaler; a comparison unit configured to compare the at least one index value with a threshold value; a creation unit configured to create stress state information based on the at least one index value based on a comparison result of the comparison unit; a selection unit configured to select at least one recommendation according to the at least one index value; and a display control unit configured to control display of the stress state information created by the creation unit and the at least one recommendation selected by the selection unit.

An information processing method according to an aspect of the present invention includes: acquiring at least one index value based on biometric data measured by a flavor inhaler; comparing the at least one index value with a threshold value; creating stress state information based on the at least one index value based on a comparison result; selecting at least one recommendation according to the at least one index value; and controlling display of the created stress state information and the at least one selected recommendation.

A program according to an aspect of the present invention causes a computer to realize: an acquisition unit configured to acquire at least one index value based on biometric data measured by a flavor inhaler; a comparison unit configured to compare the at least one index value with a threshold value; a creation unit configured to create stress state information based on the at least one index value based on a comparison result of the comparison unit; a selection unit configured to select at least one recommendation according to the at least one index value; and a display control unit configured to control display of the stress state information created by the creation unit and the at least one recommendation selected by the selection unit.

In the present invention, the "unit" or "device" does not simply refer to a physical means, but encompasses a case where functions equipped in the "unit" or "device" are implemented by software. The functions equipped in a single "unit" or "device" may be implemented by two or more physical means or devices, and the functions of two or more "units" or "devices" may be implemented by a single physical means or device.

### ADVANTAGEOUS EFFECTS OF INVENTION

According to the present invention, it is possible to provide a technique that can recommend a user of a device capable of collecting predetermined data to perform an appropriate action for stress relief.

### BRIEF DESCRIPTION OF THE DRAWINGS

FIG. 1 is a block diagram schematically showing a configuration example of an information distribution system according to a first embodiment of the present invention.
FIG. 2 is a schematic diagram schematically showing a first configuration example of an inhaling device according to the first embodiment of the present invention.
FIG. 3 is a schematic diagram schematically showing a second configuration example of the inhaling device according to the first embodiment of the present invention.
FIG. 4 is a block diagram schematically showing a configuration example of a user terminal according to the first embodiment of the present invention.
FIG. 5 is a block diagram schematically showing a configuration example of an information processing server according to the first embodiment of the present invention.
FIG. 6 is a diagram schematically showing a first configuration example of log information DB according to the first embodiment of the present invention.
FIG. 7 is a diagram schematically showing a second configuration example of the log information DB according to the first embodiment of the present invention.
FIG. 8 is a diagram schematically showing a third configuration example of the log information DB according to the first embodiment of the present invention.
FIG. 9 is a diagram schematically showing a configuration example of base data DB according to the first embodiment of the present invention.
FIG. 10 is a flowchart showing an example of base data processing by the information processing server according to the first embodiment of the present invention.
FIG. 11 is a flowchart showing an example of processing a recommendation by the information processing server according to the first embodiment of the present invention.
FIG. 12 is a diagram schematically showing a display example of a display unit of the user terminal according to the first embodiment of the present invention.
FIG. 13 is a diagram schematically showing a display example of a display unit of the user terminal according to the first embodiment of the present invention.
FIG. 14 is a diagram schematically showing a display example of a display unit of the user terminal according to the first embodiment of the present invention.
FIG. 15 is a diagram schematically showing a display example of a display unit of the user terminal according to the first embodiment of the present invention.
FIG. 16 is a diagram schematically showing a display example of a display unit of the user terminal according to the first embodiment of the present invention.
FIG. 17 is a diagram schematically showing a display example of a display unit of the user terminal according to the first embodiment of the present invention.
FIG. 18 is a block diagram schematically showing a configuration example of a user terminal according to a second embodiment of the present invention.
FIG. 19 is a diagram schematically showing a configuration example of log information DB according to the second embodiment of the present invention.
FIG. 20 is a diagram schematically showing a configuration example of base data DB according to the second embodiment of the present invention.
FIG. 21 is a flowchart showing an example of processing base data according to the second embodiment of the present invention.
FIG. 22 is a flowchart showing an example of processing a recommendation by the user terminal according to the second embodiment of the present invention.

### DETAILED DESCRIPTION

### [First Embodiment]

Hereinafter, a first embodiment will be described with reference to the accompanying drawings. In the drawings, the same constituent elements will be assigned identical reference symbols where possible, and redundant descriptions will be omitted.

### <Configuration Example of Information Distribution System>>

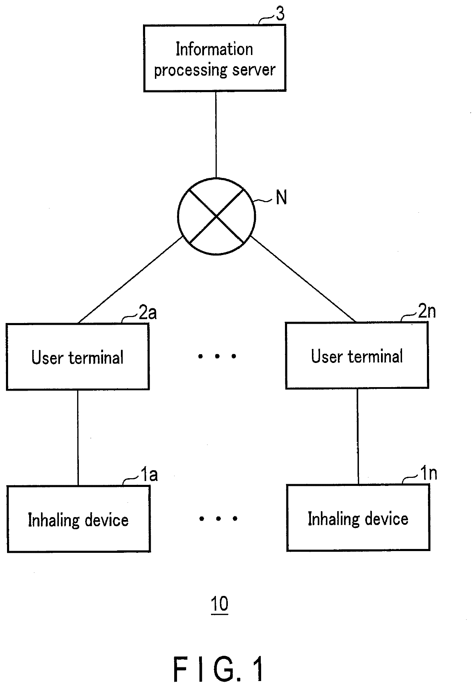

FIG. 1 is a schematic configuration diagram of an information distribution system according to the first embodiment of the present invention.

As shown in FIG. 1, an information distribution system 10 includes, as an example, n inhaling devices 1a to 1n (where n is a given integral value equal to or greater than 1), n user terminals 2a to 2n respectively connected to the n inhaling devices 1a to 1n (where n is a given integral value equal to or greater than 1), and an information processing server 3 configured to enable communication with the user terminals 2a to 2n via a network N. In the description that follows, when the n inhaling devices are not distinguished from one another, each one will be simply referred to as an "inhaling device 1", with part of the reference symbol omitted. Similarly, when the n user terminals are not distinguished from one another, each one of them will be simply referred to as a "user terminal 2", with part of the reference symbol omitted. It is assumed that an inhaling device 1 and a user terminal 2 to which the same alphabetical symbol is appended at the end are possessed by an identical user. For example, an inhaling device 1a and a user terminal 2a are possessed by an identical user.

The information distribution system 10 is what is known as a "client server system". The information distribution system 10 is realized by mutual communications between the n user terminals 2, which are clients, and the information processing server 3 via the network N. The network N is realized by, for example, the Internet, a network such as a mobile telephone network, a local area network (LAN), or a network that is a combination thereof.

The inhaling device 1 is an electronic device that produces a substance to be inhaled by a user. The inhaling device 1 corresponds to a flavor inhaler. A configuration example of an inhaling device that may be the inhaling device 1 will be described later.

A user terminal 2 is a portable electronic device equipped with a communication function. For example, the user terminal 2 is a smartphone, a tablet terminal, or the like. A configuration example of the user terminal 2 will be discussed later.

The information processing server 3 is a computer equipped with an information processing function. The information processing server 3 is realized by, for example, one or more (at least one) server devices. A configuration example of the information processing server 3 will be described later. The information processing server 3 is an example of an information processing device.

The inhaling device 1 and the user terminal 2 are associated with each other, and mutual data transmission and reception are enabled by executing short-range wireless communications based on, for example, Bluetooth (registered trademark), Bluetooth Low Energy (BLE), etc. Data transmission and reception between the inhaling device 1 and the user terminal 2 may be executed by any communication technology such as Wi-Fi (registered trademark), a low-power wide-area network (LPWAN), a near-field communication (NFC), or the like, aside from BLE communication technology. Also, data transmission and reception between the inhaling device 1 and the user terminal 2 is not necessarily performed by wireless communications, and may be performed by wired communications using a Universal Serial Bus (USB), a Mini USB, a Micro USB, Lightning, or the like.

### «Configuration Example of Inhaling Device»

An inhaling device is a device that produces a substance to be inhaled by a user. In the description that follows, it is assumed that the substance produced by the inhaling device is an aerosol. In another case, the substance produced by the inhaling device may be a gas.

### (1) First Configuration Example

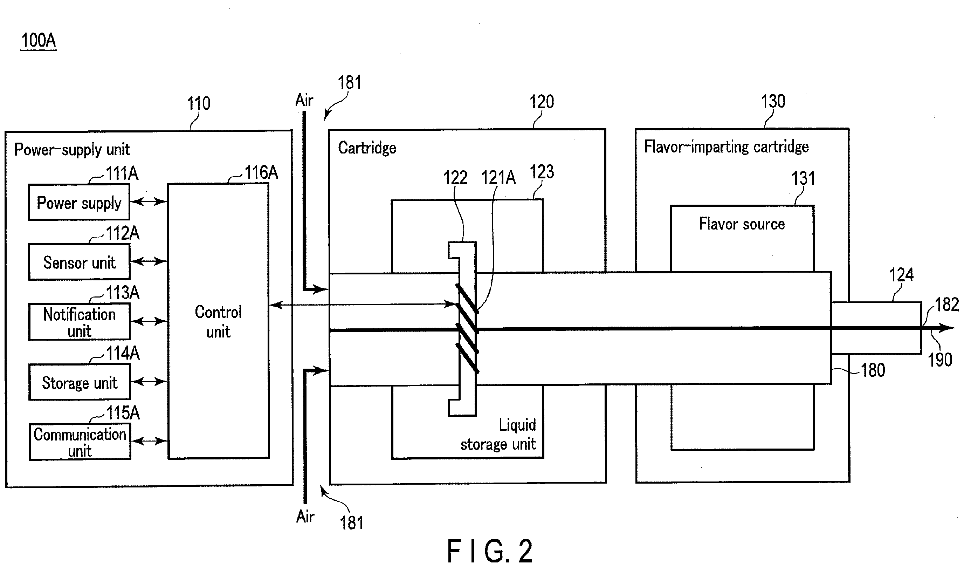

FIG. 2 is a schematic diagram schematically showing a first configuration example of the inhaling device. As shown in FIG. 2, an inhaling device 100A according to the present configuration example includes a power-supply unit 110, a cartridge 120, and a flavor-imparting cartridge 130. The power-supply unit 110 includes a power supply 111A, a sensor unit 112A, a notification unit 113A, a storage unit 114A, a communication unit 115A, and a control unit 116A. The cartridge 120 includes a heating unit 121A, a liquid guide unit 122, and a liquid storage unit 123. The flavor-imparting cartridge 130 includes a flavor source 131 and a mouthpiece 124. In the cartridge 120 and the flavor-imparting cartridge 130, an airflow path 180 is formed.

The power supply 111A accumulates electric power. Based on control by the control unit 116A, the power supply 111A supplies power to each constituent element of the inhaling device 100A. The power supply 111A may be configured of, for example, a chargeable battery such as a lithium-ion secondary battery.

The sensor unit 112A acquires various types of information relating to the inhaling device 100A. As an example, the sensor unit 112A is configured of a vital sensor that measures biometric data, a pressure sensor such as a microphone capacitor, a flowrate sensor, a temperature sensor, etc., and acquires values involved with user inhalation. As another example, the sensor unit 112A is configured of an input device which receives an information input from the user, such as a button, a switch, etc.

The notification unit 113A notifies the user of the information. The notification unit 113A is configured of, for example, a light-emitting device which emits light, a display device which displays an image, a sound output device which outputs sound, a vibration device which vibrates, etc.

The storage unit 114A stores various types of information for operation of the inhaling device 100A. The storage unit 114A is configured of, for example, a non-volatile storage medium such as a flash memory.

The communication unit 115A is a communication interface that enables communications compatible with a given wired or wireless communication standard to be performed. As such a communication standard, Wi-Fi (registered trademark), Bluetooth (registered trademark), etc. may be adopted.

The control unit 116A functions as an arithmetic processor and a controller, and controls the entire operation of the inhaling device 100A in accordance with various programs. The control unit 116A is realized by, for example, electronic circuitry such as a central processing unit (CPU), or a microprocessor.

The liquid storage unit 123 stores an aerosol source. By atomizing the aerosol source, aerosol is produced. The aerosol source is, for example, a polyhydric alcohol such as glycerin and propylene glycol, and a liquid such as water. The aerosol source may contain a tobacco-derived or non-tobacco-derived flavor component. In a case where the inhaling device 100A is a medical aspirator such as a nebulizer, the aerosol source may contain a drug.

The liquid guide unit 122 guides the aerosol source which is a liquid stored in the liquid storage unit 123 from the liquid storage unit 123, and holds the aerosol source. The liquid guide unit 122 is, for example, a wick formed by twisting a fiber material such as glass fiber or a porous material such as a porous ceramic. In this case, the aerosol source stored in the liquid storage unit 123 is guided by the capillary effect of the wick.

The heating unit 121A heats the aerosol source, atomizes the aerosol source and produce aerosol. In the example shown in FIG. 2, the heating unit 121A is configured as a coil, and is wound around the liquid guide unit 122. When the heating unit 121A generates heat, the aerosol source held in the liquid guide unit 122 is heated and atomized, thus producing aerosol. The heating unit 121A generates heat when fed from the power supply 111A. As an example, feeding may be performed when the sensor unit 112A has detected the commencement of inhaling by the user and/or the input of predetermined information. Feeding may be stopped when the sensor unit 112A has detected the termination of inhaling by the user and/or the input of predetermined information.

The flavor source 131 is a constituent element for imparting a flavor component to aerosol. The flavor source 131 may contain a tobacco-derived or non-tobacco-derived flavor component.

The airflow path 180 is a path of air inhaled by the user. The airflow path 180 has a tubular structure including, at one of both ends, an air inflow hole 181, which is an inlet of air flowing to the airflow path 180, or an air outflow hole 182, which is an outlet of air flowing from the airflow path 180. In the middle of the airflow path 180, the liquid guide unit 122 is arranged on an upstream side (the side closer to the air inflow hole 181), and a flavor source 131 is arranged on a downstream side (the side closer to the air outflow hole 182). The air flowing in from the air inflow hole 181 in accordance with user inhalation is mixed with the aerosol produced by the heating unit 121A, and, as shown by an arrow 190, passes through the flavor source 131 and is transported to the air outflow hole 182. When a mixed fluid of the aerosol and air passes through the flavor source 131, a flavor component included in the flavor source 131 is imparted to the aerosol.

The mouthpiece 124 is a member which comes in contact with the user's mouth during inhaling. In the mouthpiece 124, the air outflow hole 182 is arranged. The user can introduce a mixed fluid of the aerosol and air into the oral cavity through inhalation via the mouthpiece 124 put in the user's mouth.

A configuration example of the inhaling device 100A has been described above. As a matter of course, the configuration of the inhaling device 100A is not limited to the above, and various configurations that will be described below as examples may be adopted.

As an example, the inhaling device 100A may not include a flavor-imparting cartridge 130. In this case, a mouthpiece 124 is provided in the cartridge 120.

As another example, the inhaling device 100A may include a plurality of types of aerosol sources. Still other types of aerosol may be produced by a chemical reaction caused by a plurality of types of aerosol produced by a plurality of types of aerosol sources being mixed in the airflow path 180.

Moreover, the means for atomizing the aerosol source are not limited to heating by the heating unit 121A. For example, the aerosol source may be atomized by means such as vibration atomizing or induction heating.

### (2) Second Configuration Example

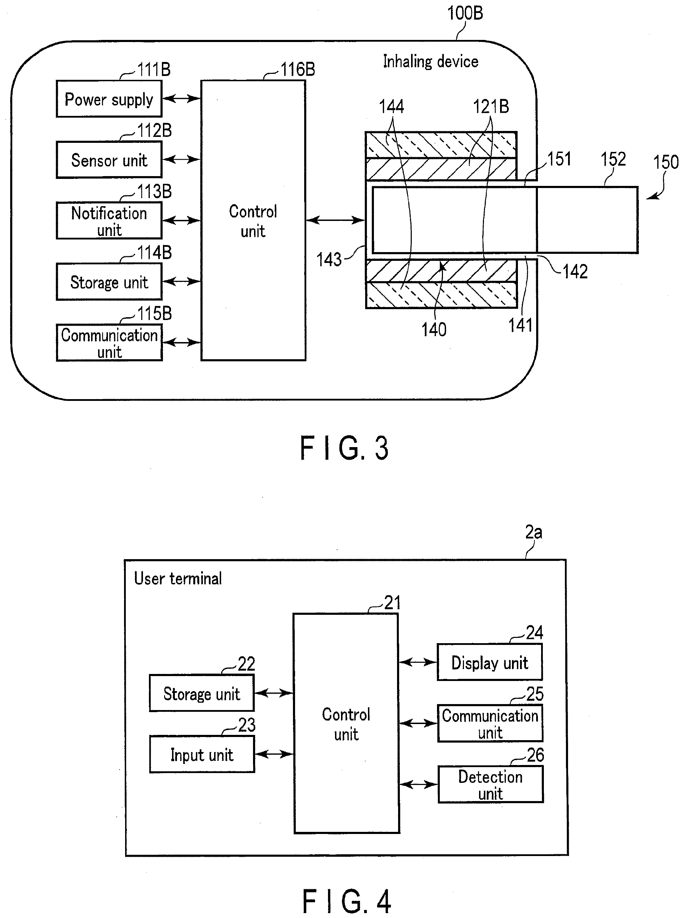

FIG. 3 is a schematic diagram schematically showing a second configuration example of the inhaling device. As shown in FIG. 3, an inhaling device 100B according to the present configuration example includes a power supply 111B, a sensor unit 112B, a notification unit 113B, a storage unit 114B, a communication unit 115B, a control unit 116B, a heating unit 121B, a holding part 140, and a heat-insulating part 144.

The power supply 111B, the sensor unit 112B, the notification unit 113B, the storage unit 114B, the communication unit 115B, and the control unit 116B are substantially identical to the corresponding constituent elements included in the inhaling device 100A according to the first configuration example.

The holding part 140 includes an internal space 141, and holds a stick-shaped base material 150 with part of the stick-shaped base material 150 housed in the internal space 141. The holding part 140 includes an opening 142 which allows the internal space 141 to be communicated to the outside, and holds the stick-shaped base material 150 inserted into the internal space 141 from the opening 142. The holding part 140 is, for example, a tubular body including the opening 142 and a bottom portion 143 as bottom surfaces, and defines a columnar internal space 141. The holding part 140 is also equipped with a function of defining a path of air that is supplied to the stick-shaped base material 150. An air inflow hole which is an inlet of air to such a path is arranged in, for example, the bottom portion 143. On the other hand, an air outflow hole which is an outlet of air from such a path is the opening 142.

The stick-shaped base material 150 includes a base material portion 151 and a suction portion 152. The base material portion 151 includes an aerosol source. In the present configuration example, the aerosol source is not limited to a liquid, and may be a solid. With the stick-shaped base material 150 held in the holding part 140, at least part of the base material portion 151 is housed in the internal space 141, and at least part of the suction portion 152 projects from the opening 142. Through user inhalation via the suction portion 152 projecting from the opening 142 which comes into contact with the user's mouth, air flows in from an air inflow hole (not illustrated) to the internal space 141, and reaches the user's oral cavity together with the aerosol produced from the base material portion 151.

The heating unit 121B has a configuration similar to that of the heating unit 121A according to the first configuration example. However, in the example shown in FIG. 3, the heating unit 121B is configured in a film shape, and is arranged so as to cover the outer periphery of the holding part 140. When the heating unit 121B generates heat, the base material portion 151 of the stick-shaped base material 150 is heated from the outer periphery, and thereby aerosol is produced.

The heat-insulating part 144 prevents heat transfer from the heating unit 121B to another constituent element. For example, the heat-insulating part 144 is configured from a vacuum-insulating material, an aerogel-insulating material, etc.

A configuration example of the inhaling device 100B has been described above. As a matter of course, the configuration of the inhaling device 100B is not limited to the above, and various configurations that will be described below as examples may be adopted.

As an example, the heating unit 121B may be configured in a blade shape and arranged so as to project from the bottom portion 143 of the holding part 140 into the internal space 141. In this case, the blade-shaped heating unit 121B is inserted into the base material portion 151 of the stick-shaped base material 150, and the base material portion 151 of the stick-shaped base material 150 is heated from the inside. As another example, the heating unit 121B may be arranged so as to cover the bottom portion 143 of the holding part 140. Also, the heating unit 121B may be configured as a combination of two or more of a first heating unit that covers an outer periphery of the holding part 140, a blade-shaped second heating unit, and a third heating unit that covers a bottom portion 143 of the holding part 140.

As another example, the holding part 140 may include an open/close mechanism such as a hinge, which opens and closes part of an outer shell that forms the internal space 141. By opening and closing the outer shell, the holding part 140 may hold the stick-shaped base material 150 inserted into the internal space 141. In this case, the heating unit 121B may be provided in the place of the holding part 140 which holds the stick-shaped base material 150, and may heat the stick-shaped base material 150 while pressing it.

Moreover, the means for atomizing the aerosol source are not limited to heating by the heating unit 121B. For example, the means for atomizing the aerosol source may be based on induction heating.

Also, the inhaling device 100B may further include the heating unit 121A, the liquid guide unit 122, the liquid storage unit 123, and the airflow path 180 according to the first configuration example, and the air outflow hole 182 of the airflow path 180 may also function as an air inflow hole to the internal space 141. In this case, a mixed fluid of the aerosol and air produced by the heating unit 121A flows into the internal space 141, is further mixed with the aerosol produced by the heating unit 121B, and reaches the oral cavity of the user.

### <<Configuration Example of User Terminal>>

FIG. 4 is a block diagram schematically showing a configuration example of a user terminal according to an embodiment of the present invention.

The user terminal 2a includes a control unit 21, a storage unit 22, an input unit 23, a display unit 24, a communication unit 25, and a detection unit 26. These units are mutually connected via a bus line.

The control unit 21 controls the entire operation of the user terminal 2a in accordance with a program stored in the storage unit 22. The control unit 21 is configured of, for example, electronic circuitry such as a processor. The processor is, for example, a CPU or the like.

The storage unit 22 is configured of a main storage and an auxiliary storage. The main storage is configured of, for example, a volatile memory that provides a work area for the processor. The main storage is configured of, for example, a random access memory (RAM) or the like. The auxiliary storage is configured of, for example, a non-volatile memory that stores various types of information and programs for operation of the user terminal 2a. The auxiliary storage is configured of, for example, a hard disk drive (HDD), a solid-state drive (SSD), or the like.

Based on the user's operation, the input unit 23 receives an instruction. The input unit 23 is configured of, for example, a keyboard, a touchpad, or the like.

The display unit 24 displays a variety of screens. The display unit 24 is configured of, for example, a liquid crystal display.

The communication unit 25 is configured of one or more communication interfaces that enable communications compatible with a given wired or wireless communication standard to be performed. The communication unit 25 includes one or more communication interfaces that enable communications to be performed between the user terminal 2a and the inhaling device 1a, as described above. The communication unit 25 includes one or more communication interfaces that enable communications to be performed between the user terminal 2a and the information processing server 3 via the network N.

The detection unit 26 is configured of a sensor that detects various types of information. The detection unit 26 includes a sensor that detects position information on the user terminal 2a. For example, the position information is information indicating latitude and longitude. For example, the sensor that detects the position information is a global positioning system (GPS) sensor.

The above-described user terminal 2a transmits user data, to be described below as an example, to the information processing server 3 based on an activation of the inhaling device 1a. For example, the user data includes biometric data and time information. The user data may include, in addition to the biometric data and the time information, some or all of the position information on the user terminal 2a and the user information of the user who owns the user terminal 2a. Based on the activation of the inhaling device 1a, the user terminal 2a receives, from the inhaling device 1a, the biometric data measured by the inhaling device 1a via the communication unit 25. In an example, the inhaling device 1a begins measurement of the biometric data based on the activation of the inhaling device 1a in response to inhalation of the inhaling device 1a by the user, and transmits the measured biometric data to the user terminal 2a. In another example, the inhaling device 1a begins measurement of the biometric data based on an input of the activation by the user using a button, a switch, r the like of the inhaling device 1a, and transmits the measured biometric data to the user terminal 2a. The connection for communication between the user terminal 2a and the inhaling device 1a may be either constantly or intermittently enabled based on the activation of the inhaling device 1a. The user terminal 2a accordingly stores the acquired biometric data in the storage unit 22.

Here, an example of biometric data measured in the inhaling device 1a will be described. The biometric data is data that can be measured in the inhaling device 1a using a known technique with respect to indexes, such as pulses, perspiration, salivary components, heartbeats, respiration, words, and a grip strength. The data relating to salivary components may include data such as concentrations relating to amylase (u/L), serotonin (µg/mL), cotisol (Pmol/mL), nitric oxide metabolite (µg/g) (salivary nitrate ion). The measurement data related to salivary components may include data such as volatile sulfur compounds (ppb) (hydrogen sulfide, methyl mercaptan, and dimethyl sulfide), acidity (pH), buffer capacity (pH), white blood cells (cells/µL), protein (mg/mL), ammonia (µg/mL), and viscosity (cp) in addition to the above-mentioned data. The data related to respiration may include a puff amount, a puff interval, a time until expiration, and the like. The biometric data is not limited to the above, and may be any biometric data of an index related to measurement of stress. The biometric data may be measured at any timing such as before smoking (before use of the inhaling device la) and after smoking (after use of the inhaling device 1a) as well as at a timing while the user is smoking through use of the inhaling device 1a (during use of the inhaling device la).

Subsequently, based on receiving the biometric data, the user terminal 2a acquires position information on the user terminal 2a. The user terminal 2a acquires the position information on the user terminal 2a detected by the detection unit 26. The position information on the user terminal 2a corresponds to position information on the user who possesses the user terminal 2a and position information on the inhaling device 1a owned by the user. The user terminal 2a stores the acquired position information in the storage unit 22 accordingly in association with the biometric data.

Based on receiving the biometric data, the user terminal 2a acquires, from the storage unit 22, the user information of the user who possesses the user terminal 2a. The user information is information unique to the user who possesses the user terminal 2a. For example, the user information is a preset user ID, user attribute information, user preference information, and the like. The user ID is an identifier uniquely assigned to the user terminal 2. The attribute information is information indicating user attributes, such as gender and age. The preference information is information indicating user preferences, such as those relating to taste. The attribute information and the preference information are input from the input unit 23 by the user. The preference information may be suitably updated.

The user terminal 2a acquires time information based on receipt of the biometric data. The user terminal 2a may acquire time information indicating a time when the biometric data has been received from the inhaling device 1a as an activation time of the inhaling device 1a. The user terminal 2a stores times information accordingly in the storage unit 22 in association with the biometric data.

Next, the user terminal 2a transmits the user data including at least the biometric data and the position information to the information processing server 3 in real time via the communication unit 25. The user data may include type information indicating the type of the inhaling device 1a. The type information may be stored in the user terminal 2a, or transmitted from the inhaling device 1a to the user terminal 2a along with the biometric data. The type information is, for example, information such as the model number of the inhaling device which indicates the difference in heating temperature. The user data may include other information. The timing at which the user terminal 2a transmits the user data to the information processing server 3 need not necessarily be in real time, and may be every predetermined period (e.g., every single minute).

A hardware configuration of the user terminal 2a is not limited to the above-described configuration. The above-described constituent elements of the user terminal 2a may be suitably omitted or changed, and a new constituent element may be added to the user terminal 2a. Herein, a configuration example of the user terminal 2a has been described; however, the configuration of the user terminal 2 other than the user terminal 2a is similar to that of the user terminal 2a, and the description thereof will be omitted. Similarly to the user terminal 2a, the user terminal 2 other than the user terminal 2a may transmit user data of the user terminal 2 to the information processing server 3.

### «Configuration Example of Information Processing Server»

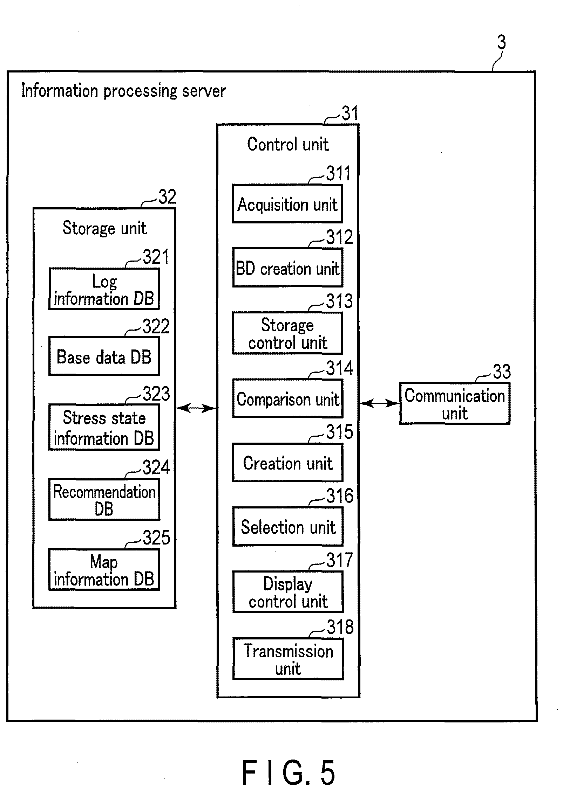

FIG. 5 is a block diagram schematically showing a configuration example of an information processing server according to the first embodiment of the present invention.

The information processing server 3 includes a control unit 31, a storage unit 32, and a communication unit 33. These units are mutually connected via a bus line.

The control unit 31 controls the entire operation of the information processing server 3 in accordance with programs stored in the storage unit 32. The control unit 31 is configured of, for example, electronic circuitry such as a processor. The processor is, for example, a CPU or the like. By executing the programs stored in the storage unit 32, the control unit 31 realizes the respective units that will be discussed later, and executes a variety of operations.

The storage unit 32 is configured of a main storage and an auxiliary storage, similarly to the above-described storage unit 22. The storage unit 32 stores programs for causing the control unit 31 to realize the respective units that will be discussed later. The storage unit 32 stores log information DB 321, base data DB 322, stress state information DB 323, recommendation DB 324, and map information DB 325.

The log information DB 321 is a database that records log information based on user data transmitted from the user terminal 2 to the information processing server 3 for each predetermined period in a time series. The log information includes a value of each index (hereinafter also referred to as an "index value") obtained based on the biometric data. The index value is a value per unit obtained based on the biometric data of each index. For example, the index value related to the pulse is a pulse rate per minute obtained based on the biometric data on the pulse. The index value is obtained by the information processing server 3 based on the biometric data. The log information DB 321 is updated every time the information processing server 3 receives user data from the user terminal 2. A configuration example of the log information DB 321 will be discussed later.

The base data DB 322 is a database that records base data created based on the log information accumulated in the log information DB 321. The base data includes a plurality of statistical values such as an average value, a maximum value, and a minimum value of the index values included in the log information. The plurality of statistical values may include a statistical value of each user associated with each user ID, a statistical value of each group obtained by grouping a plurality of users according to an attribute, a statistical value of all users within the plurality of users, and the like. Each statistical value may be a threshold value for determining the user stress state. A configuration example of the base data DB 322 will be discussed later.

The stress state information DB 323 is a database that records stress state information indicating a user stress state. The stress state information is information indicating, as a stress state, a result of comparing at least one index value of the user with a threshold value. The result of comparing the at least one index value of the user with the threshold value includes a result of comparing a stress level based on the at least one index value of the user with a stress level based on a statistical value serving as a threshold value. The stress level is a level of stress obtained by quantifying the index value and the statistical value according to a predetermined rule. The biometric data on which the index value and the statistical value are based correlates with the stress level. Therefore, the index value and the statistical value may be converted into a stress level. For example, the stress state information is text indicating a stress state such as "high stress", a graph indicating a stress state in an identifiable manner, or the like, but is not limited thereto, and may be in any format. For example, the mode in which the stress state can be identified is a mode in which an index value and a threshold value are clearly indicated. In this example, the stress state can be identified by a degree of divergence between the index value and the threshold value. For example, the mode in which the stress state can be identified is a mode in which a stress level based on an index value and a stress level based on a statistical value serving as a threshold value are clearly indicated. In this example, the stress state can be identified by the degree of divergence between the stress level based on the index value and the stress level based on the statistical value serving as the threshold value.

The recommendation DB 324 is a database that records recommendation information (hereinafter also referred to as "recommendation") to be provided to the user.

In an example, the recommendation is a recommendation based on stress state information. The recommendation based on the stress state information includes a message that recommends an action to reduce stress. For example, the recommendation based on the stress state information includes a message such as "take a break for 5 minutes" or "XX is recommended". The content of the message may be suitably updated.

In another example, the recommendation is a recommendation based on information differing from the stress state information. For example, the recommendation based on the information differing from the stress state information is a recommendation based on the position information on the user. The recommendation based on the position information on the user is a rest facility near the current location of the user. The rest facility is a facility where the user can take a rest, such as a coffee shop, a cafe, or a bookstore. Information on the rest facility may be stored either in the map information DB 325 or in the storage unit 32 in advance. The information on the rest facility may be downloaded from a server (not shown) to the information processing server 3 via the network N. The information on the rest facility may be suitably updated. The recommendation based on the information differing from the stress state information is not limited to the recommendation based on the user's position information. An example recommendation will be described later.

The map information DB 325 is a database storing map information related to an existing general map indicating states of items distributed on the ground. The map information DB 325 may include position information on each facility. The map information DB 325 may include attribute information on each facility. The attribute information is information indicating a category (bookstore, coffee shop, or the like) on each facility or an item (book, coffee, or the like) handled by each facility. The map information DB 325 may be stored in advance in the storage unit 32, or may be downloaded from a server (not illustrated) to the information processing server 3 via the network N. The map information DB 325 may be suitably updated.

The communication unit 33 is configured of one or more communication interfaces that enable communications compatible with a given wireless communication standard to be performed. The communication unit 33 includes one or more communication interfaces capable of performing communication between the information processing server 3 and the user terminal 2 via the network N as described above. For example, the communication unit 33 receives the user data from the user terminal 2 via the network N. The communication unit 33 transmits the stress state information and the at least one recommendation to the user terminal 2 via the network N.

A hardware configuration of the information processing server 3 is not limited to the above-described configuration. The above-described constituent elements of the information processing server 3 may be suitably omitted or changed, and a new constituent element may be added to the information processing server 3.

Each of the units realized by the above-described control unit 31 will be described.

The control unit 31 realizes an acquisition unit 311, a base data creation unit 312, a storage control unit 313, a comparison unit 314, a creation unit 315, a selection unit 316, a display control unit 317, and a transmission unit 318.

The acquisition unit 311 acquires, via the communication unit 33, user data from a plurality of user terminals 2 respectively connected to a plurality of inhaling devices 1, based on the respective inhaling devices 1 being activated. The acquisition unit 311 acquires at least one index value based on biometric data.

The base data creation unit 312 creates base data using log information based on the user data acquired by the acquisition unit 311. Hereinafter, the base data creation unit 312 is also referred to as a "BD creation unit 312". A configuration example of the base data will be discussed later.

The storage control unit 313 performs control so that the base data created by the BD creation unit 312 is stored in the base data DB 322.

The comparison unit 314 compares at least one index value based on the biometric data included in the user data acquired by the acquisition unit 311 with a threshold value based on the statistical value included in the base data. For example, in a case where the index value is a pulse rate, the comparison unit 314 compares the pulse rate of the user with a threshold value corresponding to an average value or the like of the pulse rates of the user included in the base data. Instead, the comparison unit 314 compares the pulse rate of the user with a threshold value corresponding to an average value or the like of pulse rates of an attribute group (for example, persons in their 30s). Instead, the comparison unit 314 compares the pulse rate of the user with a threshold value corresponding to an average value or the like of all pulse rates.

The creation unit 315 creates stress state information based on at least one index value, in turn based on the comparison result of the comparison unit 314. For example, in a case where the index value is the pulse rate of the user, the creation unit 315 determines the stress state based on the pulse rate of the user and creates the stress state information.

The selection unit 316 selects at least one recommendation in accordance with at least one index value.

The display control unit 317 controls the display of the stress state information created by the creation unit 315. The display control unit 317 controls the display of at least one recommendation selected by the selection unit 316.

The transmission unit 318 transmits a control command for controlling the inhaling device 1 in accordance with at least one index value. For example, the control command is a command for controlling a heating temperature, a smoke amount, and the like of the inhaling device 1.

### «Configuration Example of Log Information DB»

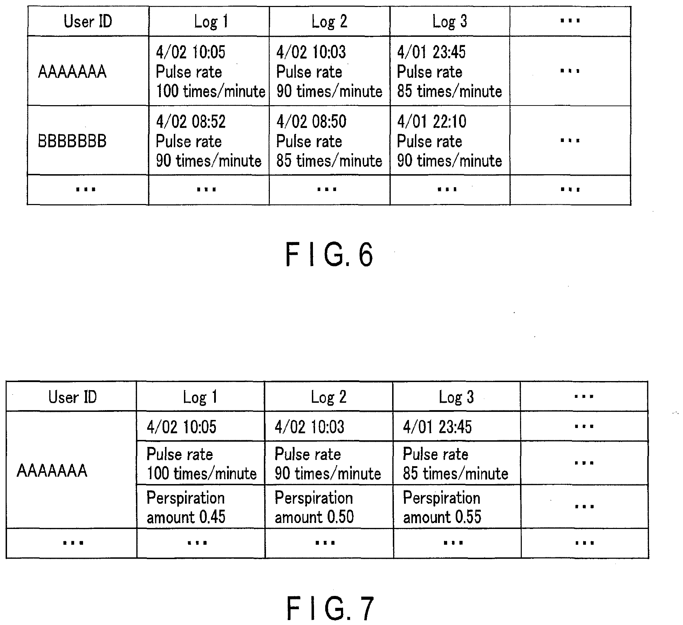

FIG. 6 is a diagram schematically showing a first configuration example of log information DB according to the embodiment of the present invention.

The log information DB 321 includes, for example, a record in which a "user ID" and log information for each predetermined period in a time series are associated with each other. Data that configures a record is configured based on user data. The "user ID" corresponds to the user ID included in the user information of the user data. The log information includes "time" and an "index value". The "time" corresponds to time information included in the user data. The "index value" corresponds to an index value obtained based on the biometric data included in the user data. In FIG. 6, the "index value" is, for example, a pulse rate, which is a value such as "pulse rate 100 times/minute". The data that configures the record may include the user attribute information and the user preference information included in the user information of the user data. The log information DB 321 may include records of a plurality of users. The log information DB 321 may be updated in response to the addition of log information.

FIG. 7 is a diagram schematically showing a second configuration example of the log information DB according to the embodiment of the present invention.

The log information DB 321 includes, for example, a record in which a "user ID" and log information for each predetermined period in a time series are associated with each other. Data that configures a record is configured based on user data. The "user ID" corresponds to the user ID included in the user information of the user data. The log information includes "time" and an "index value". The "time" corresponds to time information included in the user data. The "index value" corresponds to an index value obtained based on the biometric data included in the user data. In FIG. 7, the log information may include a plurality of index values. The "index value" includes, for example, a pulse rate and a perspiration amount, and are values such as "pulse rate 100 times/min", "perspiration amount 0.45", or the like. The data that configures the record may include the user attribute information and the user preference information included in the user information of the user data. The log information DB 321 may include records of a plurality of users. The log information DB 321 may be updated in response to the addition of log information.

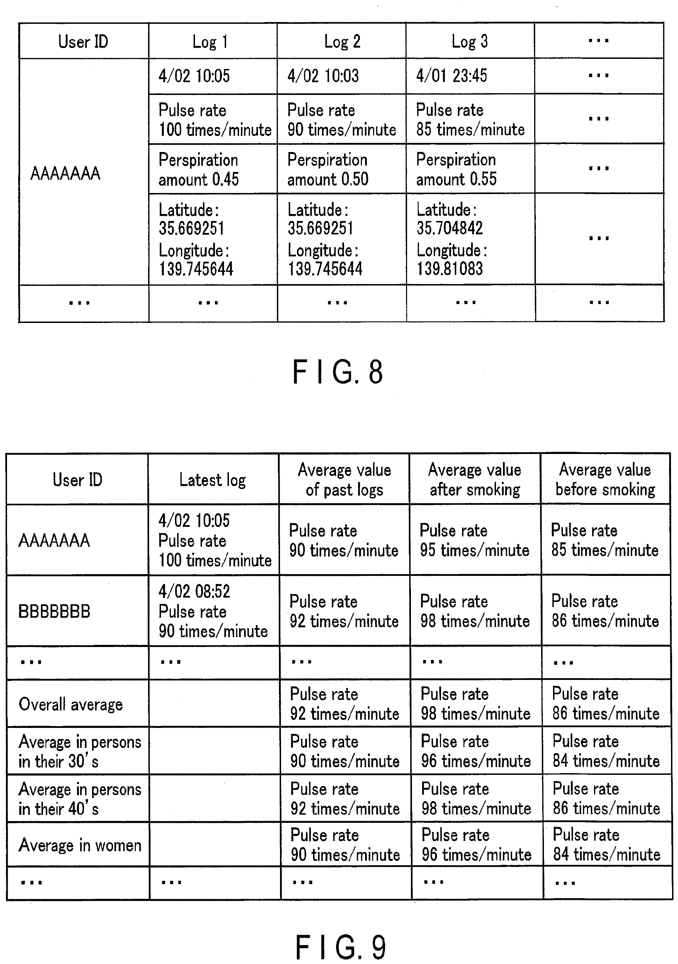

FIG. 8 is a diagram schematically showing a third configuration example of the log information DB according to the first embodiment of the present invention.

The log information DB 321 includes, for example, a record in which a "user ID" and log information for each predetermined period in a time series are associated with each other. Data that configures a record is configured based on user data. The "user ID" corresponds to the user ID included in the user information of the user data. The log information includes "time", an "index value", and a "position". The "time" corresponds to time information included in the user data. The "index value" corresponds to an index value obtained based on the biometric data included in the user data. In FIG. 8, the log information may include a plurality of index values. The "index value" includes, for example, a pulse rate and a perspiration amount, and are values such as "pulse rate 100 times/min", "perspiration amount 0.45", or the like. The "position" corresponds to position information included in the user data. The data that configures the record may include the user attribute information and the user preference information included in the user information of the user data. The log information DB 321 may include records of a plurality of users. The log information DB 321 may be updated in response to the addition of log information.

FIG. 9 is a diagram schematically showing a configuration example of base data DB according to the first embodiment of the present invention.

A "latest log" is shown for convenience and to be compared with each statistical value included in the base data; it is not included in the base data DB 322. The base data DB 322 includes, for example, a record in which a "user ID" is associated with an "average value of past logs", an "average value after smoking", an "average value before smoking", and the like. Data that configures a record is configured based on base data. The "user ID" corresponds to the user ID included in the user information of the user data. The "average value of past logs" corresponds to a statistical value based on an average of index values included in past log information for each user. The "average value after smoking" corresponds to a statistical value based on an average of index values after smoking included in the past log information for each user. The "average value before smoking" corresponds to a statistical value based on an average of index values before smoking included in the past log information for each user. The statistical value based on the average of the index values after smoking is a statistical value based on the average of the index values based on the biometric data acquired at the end time when the user ceases use of the inhaling device 1 The statistical value based on the average of the index values before smoking is a statistical value based on the average of the index values based on the biometric data acquired at the start time when the user commences use of the inhaling device 1. The base data DB 322 may include statistical values such as maximum values and minimum values after smoking in addition to the average value after smoking. The base data DB 322 may include statistical values such as maximum values and minimum values before smoking in addition to the average value before smoking. The base data DB 322 may include statistical values such as average values, maximum values, and minimum values of the amount of change before and after smoking. In a case where the base data DB 322 includes base data of a plurality of users, an "average value of past logs", an "average value after smoking", and an "average value before smoking" may include a statistical value for all users within the plurality of users and a statistical value of each group obtained by grouping the plurality of users according to an attribute. For example, the base data DB 322 may include statistical values in which items such as an "overall average", an "average in persons in their 30s", an "average in persons in their 40s", and an "average in women" are each associated with an "average value of past logs", an "average value after smoking", and an "average value before smoking". In FIG. 9, the statistical value indicates the pulse rate, but is not limited thereto, and may be the perspiration amount or the like. The base data DB 322 may include statistical values of a plurality of indexes.

### <<Example of Processing by Information Processing Server>>

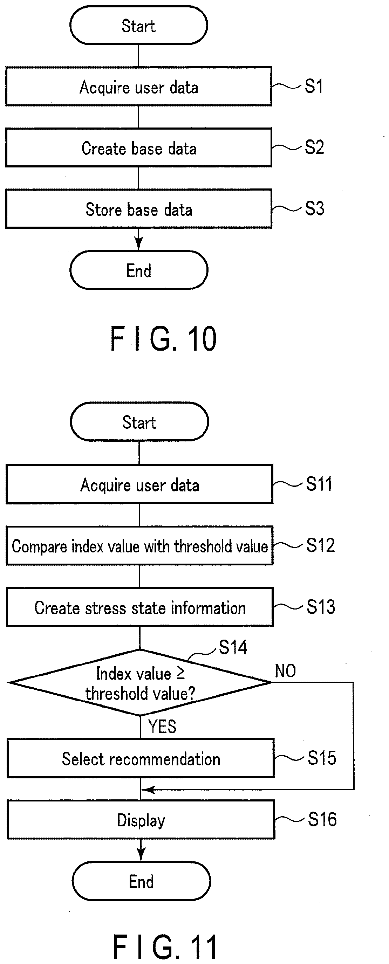

FIG. 10 is a flowchart showing an example of base data processing by the information processing server according to the first embodiment of the present invention.

The processing procedure described below is merely an example, and each act of processing may be changed as much as possibility allows. In addition, in the processing procedure described below, steps can be omitted, replaced, and added as appropriate according to the embodiment.

The acquisition unit 311 acquires user data (step S1). In step S1, for example, the acquisition unit 311 acquires the user data transmitted from the plurality of user terminals 2 via the communication unit 33, for example, in real time. The acquisition unit 311 acquires one or more index values based on the biometric data included in the user data, and acquires the one or more index values at predetermined time intervals. The acquisition unit 311 accordingly adds the log information including one or more index values to the log information DB 321 for each predetermined period in a time series. The acquisition unit 311 also accordingly adds the position information, the user information, and the time information included in the user data to the log information DB 321 as needed.

Although an example in which the user data includes biometric data has been described here, the user data is not limited thereto. In a case where the user terminal 2 can obtain one or more index values at predetermined intervals based on the biometric data, the user data may include each index value at predetermined intervals instead of the biometric data. In this case, the acquisition unit 311 acquires one or more index values based on the biometric data included in the user data. Therefore, the acquisition unit 311 need not acquire one or more index values at predetermined intervals.

The BD creation unit 312 creates the base data based on the log information stored in the log information DB 321 (step S2). In step S2, for example, the BD creation unit 312 calculates a statistical value for each user. In this example, the BD creation unit 312 calculates a statistical value such as an average value, a maximum value, or a minimum value for at least one index value associated with the user ID of each user terminal 2 included in the log information. The BD creation unit 312 may calculate a statistical value for all users within the plurality of users. The BD creation unit 312 may calculate a statistical value for each group in which a plurality of users are grouped according to an attribute.

The BD creation unit 312 may calculate a statistical value for each user, a statistical value for all users within a plurality of users, and a statistical value for each group obtained by grouping a plurality of users according to an attribute under various conditions exemplified below. For example, the BD creation unit 312 calculates a statistical value such as an average value, a maximum value, or a minimum value for each time zone. In this example, the BD creation unit 312 uses the time information included in the log information. For example, the BD creation unit 312 calculates a statistical value such as an average value, a maximum value, or a minimum value after smoking or before smoking. In this example, the BD creation unit 312 uses the time information included in the log information. The BD creation unit 312 may acquire, as the log information after smoking, the last log information among a collection of log information that continues for a certain period of time. The BD creation unit 312 may acquire, as the log information before smoking, the first log information among a collection of log information that continues for a certain period of time. For example, the BD creation unit 312 calculates a statistical value such as an average value, a maximum value, or a minimum value of the amount of change before and after smoking. In this example, the BD creation unit 312 calculates the statistical value of the amount of change before and after smoking using the statistical value before smoking and the statistical value after smoking. For example, the BD creation unit 312 calculates a statistical value such as an average value, a maximum value, or a minimum value of each user at a certain time point in a given day. In this example, the BD creation unit 312 uses the time information included in the log information.

For example, the BD creation unit 312 calculates a statistical value such as an average value, a maximum value, or a minimum value for each smoking area. In this example, the BD creation unit 312 uses the position information included in the log information. For example, the BD creation unit 312 calculates a statistical value such as an average value, a maximum value, or a minimum value for each smoking area in each time zone. In this example, the BD creation unit 312 uses the time information and the position information included in the log information. Not limited to the above, the BD creation unit 312 may calculate a statistical value by arbitrarily combining data included in the log information. The BD creation unit 312 creates base data including the various statistical values described above. Furthermore, the information processing server 3 may acquire the basic data of the user and create the base data by measuring the biometric data of the user at the time of initial use or of registering the inhaling device 1, or by measuring the biometric data of the user for a certain period such as a week.

According to this example, the information processing server 3 can compare one or more index values included in the latest log information with various thresholds. Thus, the information processing server 3 can determine the stress state of the user from various perspectives. Further, the information processing server 3 can effectively determine the stress state of the user.

The storage control unit 313 performs control so that the base data created by the base data creation unit 312 is stored in the base data DB 322 (step S3). In step S3, for example, the storage control unit 313 accordingly adds the base data created by the base data creation unit 312 to the base data DB 322.

FIG. 11 is a flowchart showing an example of processing a recommendation by the information processing server according to the first embodiment of the present invention.

The processing procedure described below is merely an example, and each act of processing may be changed as much as possibility allows. In addition, in the processing procedure described below, steps can be omitted, replaced, and added as appropriate according to the embodiment.

In order to simplify the description, the inhaling devices 1a and the user terminal 2a will be described below as examples.

The acquisition unit 311 acquires user data (step S11). In step S11, for example, the acquisition unit 311 acquires the user data transmitted from the user terminal 2a via the communication unit 33, for example, in real time. For example, the acquisition unit 311 acquires biometric data measured in the inhaling device 1a. In addition, the acquisition unit 311 acquires one or more index values based on the biometric data, and acquires one or more index values based on the biometric data. For example, the acquisition unit 311 acquires position information on the user terminal 2a connected to the inhaling device 1a. For example, the acquisition unit 311 acquires the user information from the user terminal 2a connected to the inhaling device 1a. For example, the acquisition unit 311 acquires time information from the user terminal 2a connected to the inhaling device 1a. For example, the acquisition unit 311 accordingly adds the log information including one or more index values to the log information DB 321 for each predetermined period in a time series. The acquisition unit 311 also accordingly adds the position information, the user information, and the time information to the log information DB 321 as needed.

Although an example in which the user data includes biometric data has been described here, the user data is not limited thereto. In a case where the user terminal 2a can obtain one or more index values at predetermined intervals based on the biometric data, the user data may include each index value at predetermined intervals instead of the biometric data. In this case, the acquisition unit 311 acquires one or more index values based on the biometric data included in the user data. Therefore, the acquisition unit 311 need not obtain one or more index values at predetermined intervals.

The comparison unit 314 compares at least one index value acquired by the acquisition unit 311 with the threshold value (step S12). In step S12, for example, the comparison unit 314 compares the at least one index value acquired by the acquisition unit 311 and included in the latest log information with the threshold value. The comparison unit 314 may compare at least one index value included in the latest log information with the threshold value every time the latest log information is updated. The threshold value corresponds to an arbitrary statistical value included in the base data stored in the base data DB 322. The threshold value may be a value higher than the arbitrary statistical value by a predetermined value. For example, the comparison unit 314 compares a "pulse rate" with a threshold value corresponding to a statistical value of an "average value of past logs" of pulse rates associated with the user ID of the user terminal 2a included in the base data. The comparison unit 314 may compare a stress level based on at least one index value acquired by the acquisition unit 311 with the stress level based on the statistical value serving as the threshold value.

The comparison unit 314 may compare, for each index, at least one index value with a threshold value corresponding to a statistical value associated with the user of the user terminal 2a. The comparison unit 314 may compare, for each index, at least one index value with a threshold value corresponding to a statistical value of all users within a plurality of users. The comparison unit 314 may compare, for each index, at least one index value with a threshold value corresponding to a statistical value for each group obtained by grouping according to an attribute.

The comparison unit 314 may compare, for each index, at least one index value with a plurality of threshold values corresponding to a plurality of statistical values included in the base data. For example, the comparison unit 314 compares for each index, at least one index value with threshold values respectively corresponding to the statistical values of the average value, the maximum value, and the minimum value. The comparison unit 314 may compare at least one index value included in the latest log information and acquired by the acquisition unit 311 with a threshold value corresponding to a statistical value under the same condition as the acquisition condition of the index value. For example, in consideration of at least one of the time information and the position information included in the log information, the comparison unit 314 uses a threshold value corresponding to a statistical value in consideration of at least one of the time zone and the smoking area.

The creation unit 315 creates stress state information based on at least one index value based on the comparison result of the comparison unit 314 (step S13). In step S13, for example, the creation unit 315 creates stress state information indicating a result of comparison between one index value and a threshold value as a stress state. The creation unit 315 records the created stress state information in the stress state information DB 323.

In a case where the index value is compared with one threshold value, the creation unit 315 determines the stress state based on the comparison result as exemplified below. If the index value is equal to or greater than the threshold value, the creation unit 315 may determine that the stress state is "high stress". Alternatively, if the index value is equal to or greater than the threshold value, the creation unit 315 may determine that the stress state is "medium stress" or "high stress" in accordance with the degree of divergence of the index value from the threshold value. If the index value is less than the threshold value, the creation unit 315 may determine that the stress state is "low stress".

In a case where the index value is compared with the threshold values respectively corresponding to the average value, the maximum value, and the minimum value, the creation unit 315 determines the stress state based on the comparison results as exemplified below. If the index value is less than the minimum value, the creation unit 315 may determine that the stress state is "stress level 0". If the index value is equal to or greater than the minimum value and less than the average value, the creation unit 315 may determine that the stress state is "stress level 1". If the index value is equal to or greater than the average value and less than the maximum value, the creation unit 315 may determine that the stress state is "stress level 2". If the index value is equal to or greater than the maximum value, the creation unit 315 may determine that the stress state is "stress level 3". The determination of the stress state is the same in an aspect in which a stress level based on at least one index value is compared with a stress level based on a statistical value serving as a threshold value. The creation unit 315 creates information indicating the determined stress state as stress state information. The creation unit 315 records the created stress state information in the stress state information DB 323.

In addition, the creation unit 315 may create, as the stress state information, a graph in which the relative position of the current index value of the user is superimposed on the distribution of index values. For example, in a case where the index value is compared with the statistical value of the user, the creation unit 315 creates, as the stress state information, a graph in which the relative position of the current index value of the user is superimposed on the distribution of the index values of the user. In a case where the index value is compared with the statistical value of the user, the creation unit 315 may create, as the stress state information, a graph in which the relative position of the stress level based on the current index value of the user is superimposed on the distribution of the stress levels based on the index value of the user. The creation unit 315 records the created stress state information in the stress state information DB 323.

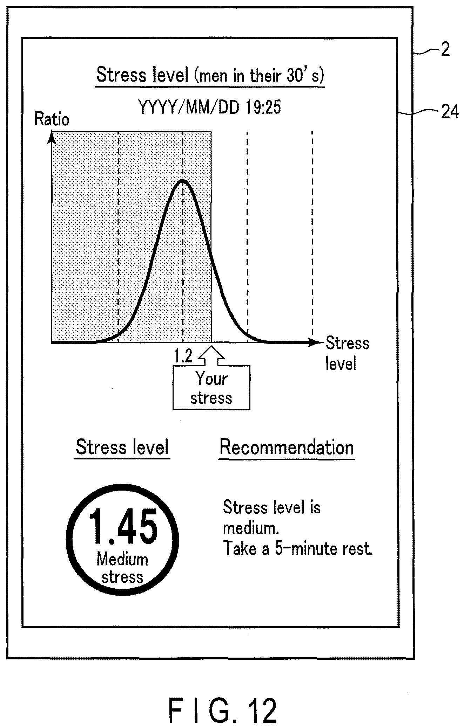

For example, in a case where the index value is compared with the statistical value of a group, the creation unit 315 creates, as the stress state information, a graph in which the relative position of the current index value of the user is superimposed on the distribution of the index values of the group. In a case where the index value is compared with the statistical value of a group, the creation unit 315 may create, as the stress state information, a graph in which the relative position of the stress level based on the current index value of the user is superimposed on the distribution of the stress levels based on the index values of the group. The creation unit 315 records the created stress state information in the stress state information DB 323. FIG. 12 is a diagram showing a display example of the display unit 24 of the user terminal 2a in a case where the current index value of the user is compared with the statistical value of "men in their 30s". According to FIG. 12, a graph showing a distribution of stress states of men in their 30s is displayed on the display unit 24 of the user terminal 2a. In the graph of the distribution of the stress states, the vertical axis represents the ratio of the number of persons, the horizontal axis represents the stress level, and the ratio of the number of persons corresponding to each stress level to the total number of persons in a group of men in their 30s is displayed as a distribution diagram. In the example of FIG. 12, the current stress level of the user is superimposed as "your stress" on the distribution diagram of the stress states of men in their 30s. The current stress level is displayed on the display unit 24 of the user terminal 2a. In the example of FIG. 12, "1.45" is displayed as the current stress level, and "medium stress" is displayed as the stress state. The current stress level is a stress level based on the current index value of the user. In this example, the average value of the stress levels of the group of men in their 30s is "1.2". The average value of the stress levels of the group of men in their 30s is a stress level based on the average value of the group of men in their 30s serving as a statistical value. The average value of the stress levels of the group of men in their 30s is clearly shown in the graph. Further, a recommendation based on the stress state information selected based on the current stress level is displayed on the display unit 24. In the example of FIG. 12, as the recommendation based on the stress state information, "The stress level is medium. Take a 5-minute rest." is displayed.

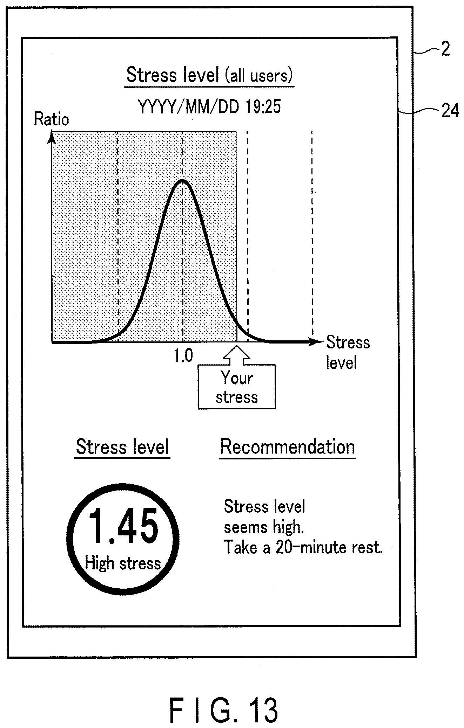

For example, in a case where the index value is compared with the statistical value of all users within the plurality of users, the creation unit 315 creates, as the stress state information, a graph in which the relative position of the current index value of the user is superimposed on the distribution of the index values for all users. In a case where the index value is compared with the statistical value of all users within the plurality of users, the creation unit 315 may create, as the stress state information, a graph in which the relative position of the current index value of the user is superimposed on the distribution of the index values for all users. The creation unit 315 records the created stress state information in the stress state information DB 323. FIG. 13 is a diagram showing a display example of the display unit 24 of the user terminal 2a in a case where the current index value of the user is compared with the statistical value for all users within the plurality of users. According to FIG. 13, a graph showing a distribution of stress states for all users is displayed on the display unit 24 of the user terminal 2a. In the graph of the distribution of the stress states, the vertical axis represents the ratio of the number of persons, the horizontal axis represents the stress level, and the ratio of the number of persons corresponding to each stress level to the total number for all users within the plurality of users is displayed as a distribution diagram. In the example of FIG. 13, the current stress level of the user is superimposed as "your stress" on the distribution diagram of the stress states for all users. The current stress level is displayed on the display unit 24 of the user terminal 2a. In the example of FIG. 13, "1.45" is displayed as the current stress level, and "high stress" is displayed as the stress state. The current stress level is a stress level based on the current index value of the user. In this example, the average value of the stress levels for all users within the plurality of users is "1.0". The average value of the stress levels for users within the plurality of users is a stress level based on the average value for all users within the plurality of users serving as a statistical value. The average value of the stress levels for all users within the plurality of users is clearly shown in the graph. Further, a recommendation based on the stress state information selected based on the current stress level is displayed on the display unit 24 of the user terminal 2a. In the example of FIG. 13, as the recommendation based on the stress state information, "The stress level seems high. Take a 20-minute rest." is displayed.

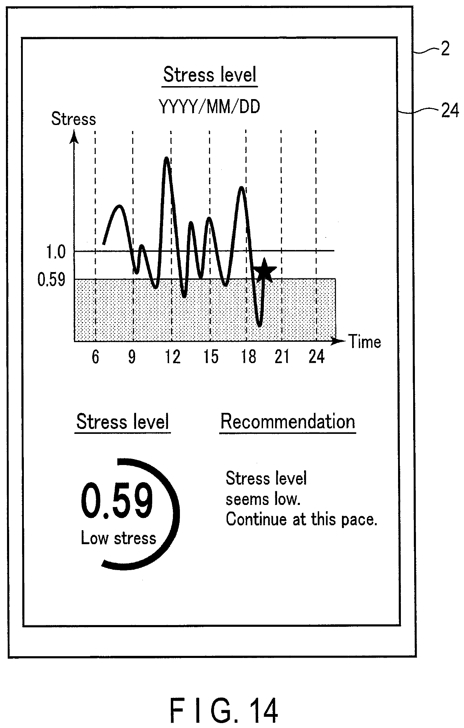

In addition, the creation unit 315 may create, as the stress state information, a graph in which the current index value is clearly indicated in the transition of the index value of the user within a given day. The creation unit 315 may create, as the stress state information, a graph in which the user stress state transition within a given day is clearly indicated. The stress state transition is a transition of the stress level based on the index value. The creation unit 315 records the created stress state information in the stress state information DB 323. FIG. 14 is a diagram showing a display example of the display unit 24 of the user terminal 2a for a graph that clearly shows a user stress state transition within a given day. According to FIG. 14, a graph showing the user stress state transition within a given day is displayed on the display unit 24 of the user terminal 2a. In the graph of the stress state transition, the vertical axis represents the stress level, the horizontal axis represents the time, and the stress level corresponding to each time is displayed as a smooth line graph. In the example of FIG. 14, the current stress level of the user is superimposed on the graph with a star sign. The current stress level is displayed on the display unit 24 of the user terminal 2a. In the example of FIG. 14, "0.59" is displayed as the current stress level, and "low stress" is displayed as the stress state. The current stress level is a stress level based on the current index value of the user. In this example, the average value of the stress levels of the user in the past day is "1.0". The average value of the past stress levels of the user is an average value of stress levels based on the past index values of the user. The average value of the past stress levels of the user is clearly shown in the graph. Further, a recommendation based on the stress state information selected based on the current stress level is displayed on the display unit 24 of the user terminal 2a. In the example of FIG. 14, as the recommendation based on the stress state information, "The stress level seems low. Continue at this pace." is displayed.

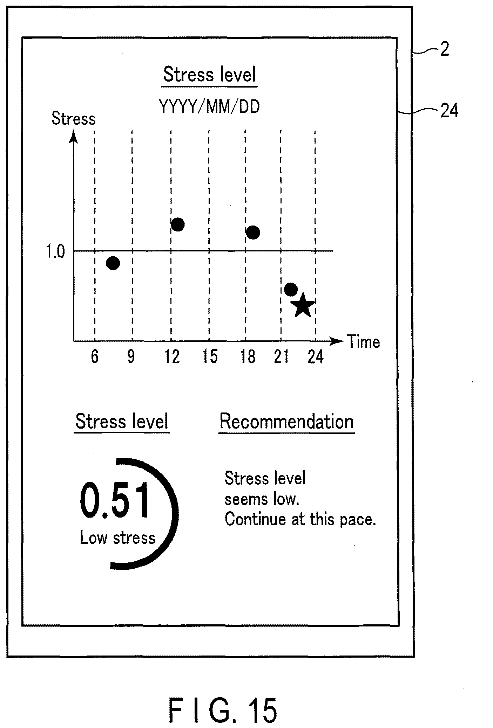

In addition, the creation unit 315 may create, as the stress state information, a graph in which the current index value is clearly indicated in the transition of the index value of the user at a predetermined timing within a given day. The creation unit 315 may create, as the stress state information, a graph that clearly indicates the user stress state transition at a predetermined timing within a given day. For example, the predetermined timing is a time of smoking. The time of smoking may be before smoking, after smoking, or both. The creation unit 315 records the created stress state information in the stress state information DB 323. FIG. 15 is a diagram showing a display example of the display unit 24 of the user terminal 2a for a graph that clearly shows a transition of the user stress state at the times of smoking within a given day. In the graph of stress state transition, the vertical axis represents the stress level, the horizontal axis represents the time, and the stress level corresponding to each timing of smoking is displayed as a plot diagram. In the example of FIG. 15, the current stress level of the user is plotted on the graph with a star sign. The current stress level is displayed on the display unit 24 of the user terminal 2a. In the example of FIG. 15, "0.51" is displayed as the current stress level, and "low stress" is displayed as the stress state. The current stress level is a stress level based on the current index value of the user. In this example, the average value of all of the past stress levels of the user is "1.0". The average value of the past stress levels of the user is an average value of stress levels based on the past index values of the user. The average value of the past stress levels of the user is clearly shown in the graph. Further, a recommendation based on the stress state information selected based on the current stress level is displayed on the display unit 24 of the user terminal 2a. In the example of FIG. 15, as the recommendation based on the stress state information, "The stress level seems low. Continue at this pace." is displayed.

The stress state information is not limited to text and graphs, and may be in any format. The determination of the stress state can be achieved using known techniques. According to this example, the information processing server 3 can provide the user stress state in an easily visible format such as text or a graph. The information processing server 3 can recommend the user to take a measure for stress relief.

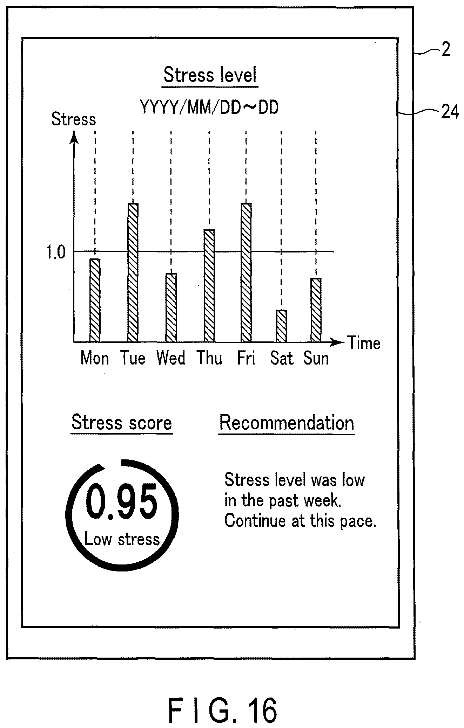

In addition, the creation unit 315 may create, as the stress state information, a graph in which the user stress state transition during a predetermined period is clearly indicated. The predetermined period of time may be, for example, one day, one week, one month, or the like. FIG. 16 is a diagram showing a display example of the display unit 24 of the user terminal 2a in a case where the predetermined period is one week. According to FIG. 16, a graph showing a transition of the user stress state within one week is displayed on the display unit 24 of the user terminal 2a. In the graph of the stress state transition, the vertical axis represents the stress level, the horizontal axis represents the time (the day of the week), and the stress level corresponding to each day of the week is displayed as a bar graph. A stress score indicating an average value of the stress levels in a given week is displayed on the display unit 24 of the user terminal 2a. In the example of FIG. 16, "0.95" is displayed as the stress score, and "low stress" is displayed as the stress state. In this example, the average value of the past stress levels of the user users is "1.0". The average value of the past stress levels of the user is clearly shown in the graph. Furthermore, a recommendation selected based on the stress score for one week is displayed on the display unit 24 of the user terminal 2a. In the example of FIG. 16, as the recommendation, "The stress level was low in the past week. Continue at this pace." is displayed.

As a result of comparing at least one index value with the threshold values in step S12, if the comparison unit 314 determines that the at least one index value is equal to or greater than at least one threshold value (step S14: YES), the process transitions from step S14 to step S15. As a result of comparing at least one index value with the threshold values in step S12, if the comparison unit 314 determines that no index value is equal to or greater than any threshold value (step S14: NO), the process transitions from step S14 to step S16.

The selection unit 316 selects at least one recommendation in response to at least one index value being equal to or greater than the threshold value (step S15). In step S15, for example, the selection unit 316 selects at least one recommendation stored in the recommendation DB 324. The selection unit 316 selects at least one recommendation in consideration of the user information acquired by the acquisition unit 311.

The selection unit 316 selects a recommendation based on the stress state information as a recommendation included in the at least one recommendation. For example, in a case where the stress state is "medium stress" or higher, or "stress level 1" or higher, the selection unit 316 selects a recommendation based on the stress state information for stress relief from the recommendation DB 324. For example, the recommendation based on the stress state information includes a recommendation for recommending a rest, a recommendation for a flavor for stress relief, a recommendation for food or drink such as coffee or soft drink, a recommendation for a browsable video or moving image, a recommendation for a game, and the like. In a specific example, the recommendation based on the stress state information is the text of a comment such as "Take a rest for 5 minutes" or "XX is recommended". The recommendation based on the stress state information may be a uniform resource locator (URL) of a service in which users whose stress state is determined to be "medium stress" or higher or "stress level 1" or higher can communicate with each other. According to this example, the information processing server 3 can select a recommendation to recommend the user to take a measure for stress relief in accordance with the user stress state. The information processing server 3 can recommend the user to take a measure for stress relief based on the selected recommendation.

In addition, the selection unit 316 may select a recommendation based on the stress state information in consideration of the user information acquired by the acquisition unit 311. For example, in a case where the user information includes user preference information, the selection unit 316 may select a recommendation of a flavor, food or drink such as coffee or confectionery, a browsable video or moving image, a game, or the like in consideration of the preference information. According to this example, the information processing server 3 can select a recommendation in consideration of the user information such as the user preference in accordance with the user stress state. The information processing server 3 can recommend each user to take a more appropriate measure for stress relief based on the selected recommendation.

In addition, the selection unit 316 may select a recommendation based on the stress state information in consideration of the type information acquired by the acquisition unit 311. For example, in a case where the user data includes type information, the selection unit 316 may select a recommendation of a flavor according to the type of the inhaling device 1a in consideration of the type information. According to this example, the information processing server 3 can select a recommendation in consideration of the type information of the inhaling device 1a in accordance with the user stress state. The information processing server 3 can recommend each user to take a more appropriate measure for stress relief based on the selected recommendation.

The selection unit 316 selects a recommendation based on information differing from the stress state information as a recommendation included in the at least one recommendation. For example, the information differing from the stress state information is position information on the user terminal 2a acquired by the acquisition unit 311. In this example, as exemplified below, the selection unit 316 selects a rest facility as a recommendation included in the at least one recommendation based on the position information on the user terminal 2a. The selection unit 316 acquires the position information on the user terminal 2a included in the latest log information. The selection unit 316 acquires the position information on rest facilities stored in the recommendation DB 324 among the position information on the respective facilities included in the map information DB 325. The selection unit 316 compares the position information on the user terminal 2a with the position information on the rest facilities. The selection unit 316 selects one or more rest facilities near the current location of the user. The number of rest facilities selected by the selection unit 316 can be set as appropriate. The range centered on the current location of the user for the selection unit 316 to select rest facilities can be set as appropriate. The selection unit 316 selects the selected one or more rest facilities as recommendations. According to this example, the information processing server 3 can select a rest facility near the current location of the user as a recommendation in accordance with the user stress state. The information processing server 3 can recommend the user to a take a measure for stress relief based on the selected recommendation, such as visiting a nearby rest facility.

In addition, the selection unit 316 may select a recommendation based on the position information in consideration of the user information acquired by the acquisition unit 311. For example, in a case where the user information includes user preference information, the selection unit 316 compares the user preference information with attribute information of the respective facilities included in the map information DB 325. The selection unit 316 may select, as a recommendation, a rest facility that matches the user preference from among one or more rest facilities near the current location of the user. For example, in a case where the preference information includes information indicating that the user likes coffee, the selection unit 316 may select a coffee shop near the current location of the user as a recommendation. According to this example, the information processing server 3 can select a recommendation in consideration of the user information, such as the user preference, and the position information in accordance with the user stress state. Based on the selected recommendation, the information processing server 3 can recommend a rest facility that is close to the current location of the user and suits the preference of the user. The information processing server 3 can recommend each user to take a more appropriate measure for stress relief.

The recommendation based on information differing from the stress state information may be information such as a positive word, the words of an eminent person, fortune-telling, news, or the like. The recommendation based on information differing from the stress state information may be the provision of points, coins, game items, or the like. In a case where the user terminal 2a is a device that emits sound, the recommendation based on information differing from the stress state information may be predetermined audio information such as a person's laughter. In a case where the user terminal 2a is a device that emits light, the recommendation based on information differing from the stress state information may be a predetermined light emission pattern. In a case where the user terminal 2a is a vibratory device, the recommendation based on information differing from the stress state information may be a predetermined vibration pattern. According to this example, the information processing server 3 can select various recommendations in accordance with the user stress state. The information processing server 3 can recommend the user to take measures for relieving various stresses based on the selected recommendations. The selection of recommendations may be performed using known machine learning techniques.

As a recommendation included in the at least one recommendation, the selection unit 316 may independently select either or both of: the recommendation based on the stress state information and the recommendation based on information differing from the stress state information.

The display control unit 317 controls display of at least the stress state information (step S16). In step S16, for example, in a case where the selection unit 316 does not select at least one recommendation, the display control unit 317 controls the display of the stress state information. In this example, the display control unit 317 transmits the stress state information to the user terminal 2a. For example, in a case where the selection unit 316 selects at least one recommendation, the display control unit 317 controls the display of the stress state information and the at least one recommendation. In this example, the display control unit 317 transmits the stress state information and the at least one recommendation to the user terminal 2a.

The display control unit 317 controls the user terminal 2a to display the stress state information created by the creation unit 315 in step S13. The user terminal 2a displays the stress state information on the display unit 24. The display of the stress state information may be, but is not limited to, text display, graph display, or the like, and may be of any display mode. According to this example, the information processing server 3 can provide the user stress state in an easily visible format. The information processing server 3 can recommend the user to take a measure for stress relief.

The display control unit 317 controls the user terminal 2a to display one or more recommendations selected by the selection unit 316 in step S15.

In a case where the selection unit 316 selects the recommendations based on the stress state information, the display control unit 317 controls the display of the recommendations based on the stress state information. The user terminal 2a displays the recommendations based on the stress state information on the display unit 24.

In a case where the selection unit 316 selects a recommendation based on the information differing from stress state information, the display control unit 317 controls the display of the recommendation based on the information differing from the stress state information. The user terminal 2a display the recommendation based on the information differing from the stress state information on the display unit 24. For example, in a case where the selection unit 316 selects a rest facility, the display control unit 317 controls the display of a map on which the rest facility is superimposed. In this example, the display control unit 317 transmits the map on which the rest facility is superimposed to the user terminal 2a. The user terminal 2a displays the map on which the rest facility is superimposed on the display unit 24.

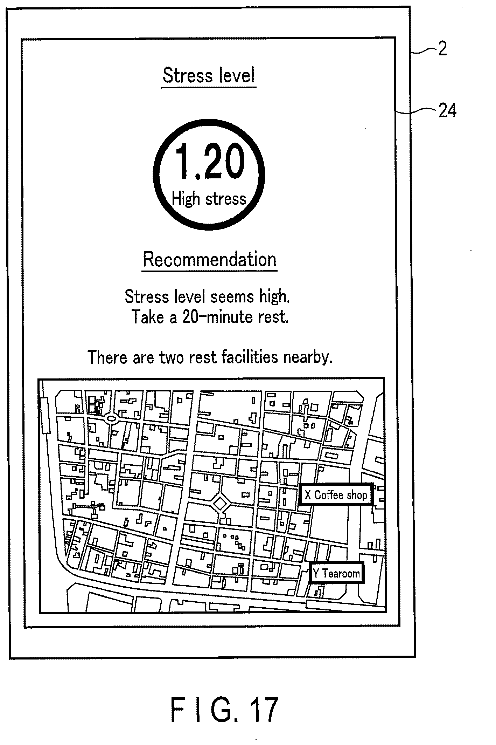

FIG. 17 is a diagram illustrating a display example of the display unit 24 of the user terminal 2a in a case where a rest facility is selected as a recommendation. In the example of FIG. 17, "1.20" is displayed as the current stress level, and "high stress" is displayed as the stress state. The current stress level is a stress level based on the current index value of the user. Further, a recommendation based on the stress state information selected based on the current stress level is displayed on the display unit 24 of the user terminal 2a. In the example of FIG. 17, as the recommendation based on the stress state information, "The stress level seems high. Take a 20-minute rest." is displayed. Further, the display unit 24 of the user terminal 2a displays the rest facility selected by the selection unit 316, based on the position information on the user terminal 2a, so as to be superimposed on the map around the current location. In the example of FIG. 17, a coffee shop and a tearoom are displayed on the map as rest facilities close to the current location of the user. In the example of FIG. 17, as the recommendation based on information differing from the stress state information, "There are two rest facilities nearby." is displayed.

The display of the at least one recommendation may be, but is not limited to text display, map display, or the like, and may be of any display mode. According to this example, the information processing server 3 can provide the recommendation in an easily visible format. The information processing server 3 can recommend the user to take a measure for stress relief.

The at least one recommendation may be displayed either on the same screen as the stress state information or on another screen. For example, the recommendation based on the stress state information may be displayed either on the same screen as the stress state information or on another screen. For example, the recommendation based on the information differing from the stress state information may be displayed either on the same screen as the stress state information or on another screen. For example, the recommendation based on the stress state information may be displayed either on the same screen as the recommendation based on information differing from the stress state information, or on another screen.

The display control unit 317 may perform appropriate control in accordance with the recommendation selected by the selection unit 316. For example, in a case where points, coins, or the like are selected as a recommendation, the display control unit 317 may control the user terminal 2a to display the number of points or coins to be given. For example, in a case where a sound, a light emission pattern, or a vibration pattern is selected as a recommendation, the display control unit 317 may control the user terminal 2a to provide them. For example, in a case where a communication service is selected as a recommendation, the display control unit 317 may control the user terminal 2a to display the URL of the service. According to this example, the information processing server 3 can provide various recommendations to the user. The information processing server 3 can recommend the user to take measures for relieving various stresses.

In a case where the inhaling device 1a includes a display unit, the display control unit 317 may control display of the stress state information and the at least one recommendation for the inhaling device 1a. In this example, the display control unit 317 transmits, to the user terminal 2a, the stress state information, the at least one recommendation, and an instruction to transfer them to the inhaling device 1a. In this case, the display mode may be the same as or different from that of the user terminal 2a. For example, the display for the inhaling device 1a may be a display of abbreviations, icons, or the like, indicating the stress state information, or abbreviations, icons, or the like, indicating the content of the recommendation. According to this example, the information processing server 3 can provide the stress state information and the recommendation at the inhaling device 1a. The information processing server 3 can increase the visibility to the user and more efficiently recommend the user to take a measure for stress relief.

In step S14, if the comparison unit 314 determines that at least one index value is equal to or greater than at least one threshold value, the transmission unit 318 may transmit a control command. The transmission unit 318 transmits a control command for controlling the inhaling device 1a to the user terminal 2a via the communication unit 33 in response to at least one index value being equal to or greater than the threshold value. In this example, the communication unit 33 transmits the control command to the user terminal 2a. The user terminal 2a forwards the control command to the inhaling devices 1a. The inhaling device 1a controls the heating temperature, the smoke amount, etc. using any known technique based on the received control commands. According to this example, if it is determined that the stress is high, the information processing server 3 can change the heating temperature, the smoke amount, and the like of the inhaling device 1a. The information processing server 3 can control the inhaling state of the user and support relief of the stress state.

### «Modification»

The first embodiment may include the following modification.

The selection unit 316 may select a recommendation based on the stress state information with the length of the rest time being changed according to the degree of divergence of at least one index value from the threshold value. The selection unit 316 may set the rest time to be longer as the degree of divergence increases. According to this example, the information processing server 3 can recommend the user to take an appropriate measure for stress relief according to the degree of the stress state.

Further, in a case where the comparison unit 314 compares at least one index value with a threshold value corresponding to the maximum value, the selection unit 316 may select at least one recommendation according to the magnitude of the at least one index value as exemplified below. For example, in response to at least one index value being equal to or greater than the maximum value, the selection unit 316 selects a recommendation based on stress state information indicating that the stress state is very high. The recommendation based on the stress state information is a warning that strongly recommends stress relief, a recommendation that recommends a rest for a length of time (for example, 20 minutes) longer than a normal length of time (for example, 5 minutes), or the like. In this example, the transmission unit 318 may transmit a control command for prohibiting the use of the inhaling device 1 until at least one index value becomes less than the maximum value. The at least one index value to be compared by the comparison unit 314 may be an index value before smoking, an index value during smoking, or an index value after smoking.

Further, in a case where the comparison unit 314 compares at least one index value with a threshold value corresponding to the minimum value, the selection unit 316 may select at least one recommendation according to the magnitude of the at least one index value as exemplified below. For example, in response to at least one index value being less than the minimum value, the selection unit 316 selects a recommendation based on stress state information indicating an abnormal value. The recommendation based on the stress state information is a warning that strongly recommends a rest, a recommendation that recommends a rest for a length of time (for example, 20 minutes) longer than a normal length of time (for example, 5 minutes), or the like. The recommendation based on the stress state information may be a recommendation for recommending a check of the state of the inhaling device 1 in order to re-measure the biometric data in the inhaling device 1. This is because there is a possibility that the inhaling device 1 does not accurately measure biometric data. In this example, the transmission unit 318 may transmit a control command for prohibiting the use of the inhaling device 1 until at least one index value become equal to or greater than the minimum value. The at least one index value to be compared by the comparison unit 314 may be an index value before smoking, an index value during smoking, or an index value after smoking.

Further, in a case where the comparison unit 314 compares at least one index value with threshold values respectively corresponding to the average value, the maximum value, and the minimum value, the selection unit 316 may select at least one recommendation according to the magnitude of the at least one index value as exemplified below. For example, the selection unit 316 selects at least one recommendation in response to the at least one index value being closer to the maximum value than the average value. This is because the comparison unit 314 determines that the user stress state is high. For example, the selection unit 316 omits selection of at least one recommendation in response to the at least one index value being closer to the minimum value than the average value. This is because the comparison unit 314 determines that the user stress state is not high.

Furthermore, in a case where the comparison unit 314 compares at least one index value related to salivary components with a threshold value, the selection unit 316 may select a recommendation for an oral cavity state determinable from the measured salivary components. For example, the selection unit 316 may select a recommendation related to breath odor and periodontal diseases.

Furthermore, when acquiring the biometric data measured by the inhaling device 1, the information processing server 3 may give points, coins, items, or the like to the user terminal 2. In addition, the information processing server 3 may change points or the like to be given in accordance with the degree of the stress state. According to this example, the information processing server 3 can be expected to increase opportunities for providing biometric data by the user, and can effectively execute creation of stress state information.

Furthermore, the information processing server 3 may select a recommendation in consideration of user schedule information by cooperating with a scheduler of the user terminal 2. According to this example, the information processing server 3 can recommend the user to take a measure for stress relief in accordance with the user schedule information.

### [Second Embodiment]

Hereinafter, a second embodiment will be described with reference to the accompanying drawings.

In the second embodiment, similarly to the first embodiment, stress state information is created based on user data, and the stress state information and a recommendation are displayed on a user terminal. Unlike the first embodiment, the second embodiment is an example in which the user terminal 2 performs the same processing as the information processing server 3. In the second embodiment, the user terminal 2 is an example of an information processing device. The same components as those of the first embodiment are denoted by the same reference numerals, and descriptions thereof are omitted. The explanation of the second embodiment will focus mainly on the portions that differ from the first embodiment.

### «Configuration Example of User Terminal»

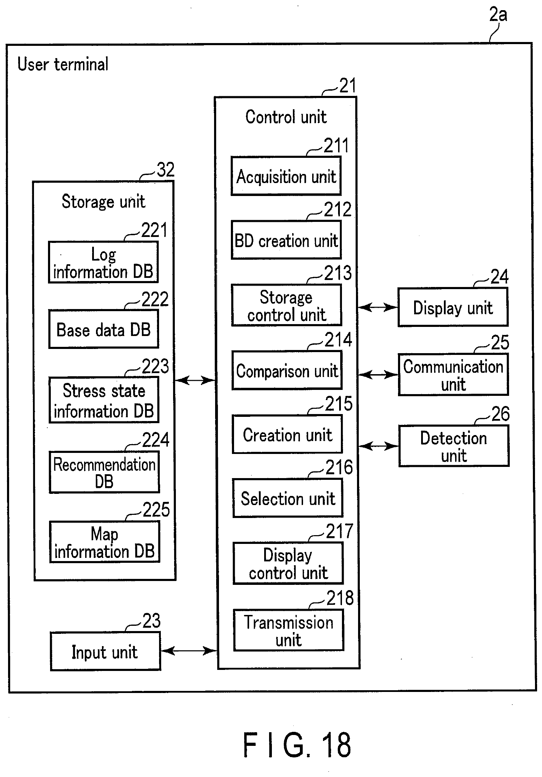

FIG. 18 is a block diagram schematically showing a configuration example of a user terminal 2a according to the second embodiment of the present invention.

The storage unit 22 stores programs for causing the control unit 21 to realize the respective units that will be discussed later. The storage unit 22 stores log information DB 221, base data DB 222, stress state information DB 223, recommendation DB 224, and map information DB 225.

The log information DB 221 is a database that records log information based on user data acquired by the user terminal 2a for each predetermined period in a time series. The log information DB 221 is updated every time user data is acquired. A configuration example of the log information DB 221 will be discussed later.

The base data DB 222 is a database that records base data created based on the log information accumulated in the log information DB 221. The base data includes a plurality of statistical values such as an average value, a maximum value, and a minimum value of the index values included in the log information of the user terminal 2a. A configuration example of the base data DB 222 will be discussed later.

The stress state information DB 223 is a database that records stress state information indicating a stress state of the user. The stress state information is the same as the stress state information of the first embodiment.

The recommendation DB 224 is a database that records recommendations to be provided to the user. The recommendations are similar to the recommendations of the first embodiment, and includes a recommendation based on the stress state information and a recommendation based on information differing from the stress state information. Information on a rest facility to be recommended may be stored either in the map information DB 225 or in the storage unit 22 in advance. The information on the rest facility may be downloaded from a server (not shown) to the user terminal 2a via the network N.

The map information DB 225 has the same configuration as the map information DB 325 of the first embodiment. The map information DB 225 may be stored in advance in the storage unit 22, or may be downloaded from a server (not illustrated) to the user terminal 2a via the network N. The map information DB 225 may be suitably updated.

A communication unit 25 receives biometric data from an inhaling device 1a.

A hardware configuration of the user terminal 2a is not limited to the above-described configuration. The above-described constituent elements of the user terminal 2a may be suitably omitted or changed, and a new constituent element may be added to the user terminal 2a.

Each of the units realized by the above-described control unit 21 will be described.

The control unit 21 realizes an acquisition unit 211, a base data creation unit 212, a storage control unit 213, a comparison unit 214, a creation unit 215, a selection unit 216, a display control unit 217, and a transmission unit 218.

The acquisition unit 211 acquires biometric data from the inhaling device 1a connected to the user terminal 2a via the communication unit 25 based on activation of the inhaling device 1a. The acquisition unit 211 accordingly stores the acquired biometric data in the storage unit 22. The acquisition unit 211 acquires the user data from the storage unit 22. For example, the acquisition unit 211 acquires the biometric data from the storage unit 22. Similarly to the acquisition unit 311, the acquisition unit 211 acquires at least one index value based on biometric data. For example, the acquisition unit 211 acquires position information associated with the biometric data from the storage unit 22. For example, the acquisition unit 211 acquires time information associated with the biometric data from the storage unit 22. For example, the acquisition unit 211 acquires user information from the storage unit 22.

Similarly to the base data creation unit 312, the base data creation unit 212 creates base data using log information based on the user data acquired by the acquisition unit 211. Hereinafter, the base data creation unit 212 is also referred to as a BD creation unit 212. A configuration example of the base data will be discussed later.

Similarly to the storage control unit 313, the storage control unit 213 performs control so that the base data created by the base data creation unit 212 is stored in the base data DB 222

Similarly to the comparison unit 314, the comparison unit 214 compares at least one index value acquired by the acquisition unit 211 with a threshold value based on a statistical value included in the base data.

Similarly to the creation unit 315, the creation unit 215 creates stress state information based on at least one index value based on the comparison result of the comparison unit 214.

Similarly to the selection unit 316, the selection unit 216 selects at least one recommendation according to at least one index value.

Similarly to the display control unit 317, the display control unit 217 controls display of the stress state information. The display control unit 217 controls display of at least one recommendation selected by the selection unit 216.

Similarly to the transmitter 318, the transmission unit 218 transmits a control command for controlling the inhaling device 1a in accordance with at least one index value.

### «Configuration Example of Log Information DB»

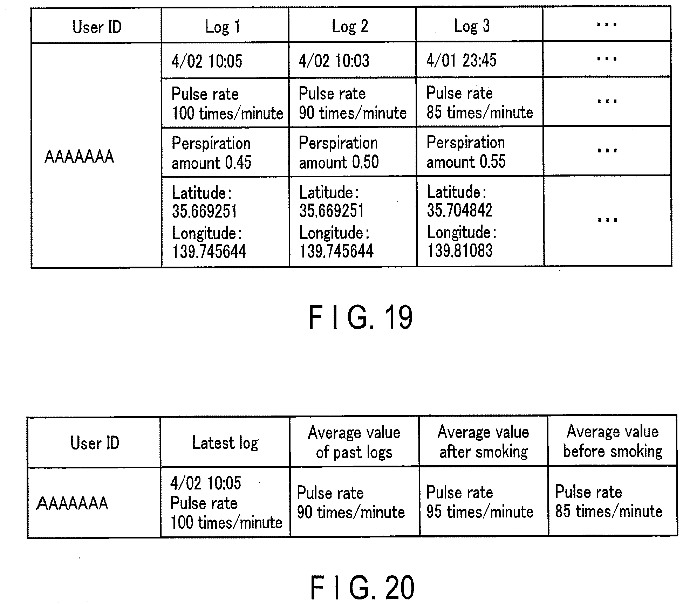

FIG. 19 is a diagram schematically showing a configuration example of log information DB according to the second embodiment of the present invention.

Similarly to the log information DB 321, the log information DB 221 includes a record in which log information and the like for each predetermined period in a time series are associated with each other. Data that configures a record is configured based on user data. The "user ID" corresponds to the user ID included in the user information of the user data. The log information includes "time" and an "index value". The "time" corresponds to time information included in the user data. The "index value" corresponds to an index value obtained based on the biometric data included in the user data. In FIG. 19, the log information may include a plurality of index values. For example, the "index value" includes, for example, a pulse rate and a perspiration amount. The "position" corresponds to position information included in the user data. The data that configures the record may include the user attribute information and the user preference information included in the user information of the user data. The log information DB 221 may be updated in response to the addition of log information.

FIG. 20 is a diagram schematically showing a configuration example of base data DB according to the second embodiment of the present invention.

A "latest log" is shown for convenience and to be compared with each statistical value included in the base data; it is not included in the base data DB 222. Similarly to the base data DB 322, the base data DB 222 includes, for example, a record in which a "user ID" is associated with an "average value of past logs", an "average value after smoking", an "average value before smoking", and the like. Data that configures a record is configured based on base data. The "user ID" corresponds to the user ID included in the user information of the user data of the user terminal 2a. The "average value of past logs" corresponds to a statistical value based on an average of index values included in past log information for the user who possesses the user terminal 2a. The "average value after smoking" corresponds to a statistical value based on an average of index values after smoking included in the past log information for the user who possesses the user terminal 2a. The "average value before smoking" corresponds to a statistical value based on an average of index values before smoking included in the past log information for the user who possesses the user terminal 2a. Similarly to the base data DB 322, the base data DB 222 may include various statistical values related to the user who possesses the user terminal 2a. In FIG. 20, the index value indicates the pulse rate, but is not limited thereto, and may be the perspiration amount or the like, or may include a plurality of index values. The base data DB 222 may include statistical values of a plurality of indexes.

### <<Example of Processing by User Terminal>>

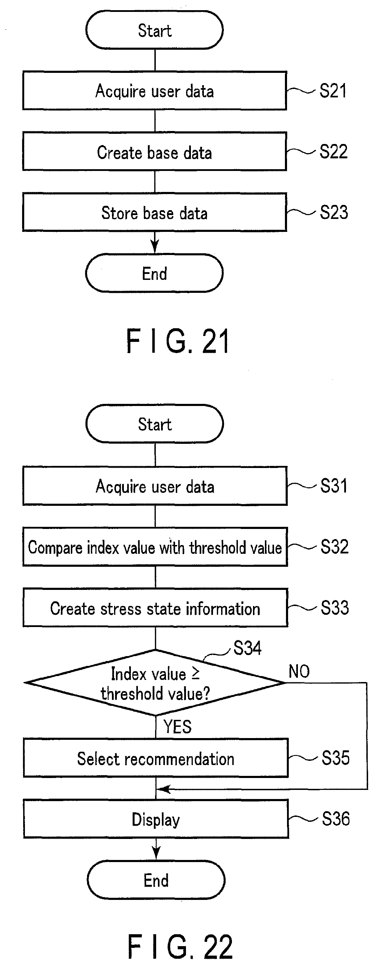

FIG. 21 is a flowchart showing an example of processing base data according to the second embodiment of the present invention.

The processing procedure described below is merely an example, and each act of processing may be changed as much as possibility allows. In addition, in the processing procedure described below, steps can be omitted, replaced, and added as appropriate according to the embodiment.

The acquisition unit 211 acquires user data (step S21). In step S21, for example, the acquisition unit 211 acquires the user data from the storage unit 22. The acquisition unit 211 acquires one or more index values based on biometric data included in the user data, and acquires the one or more index values at predetermined time intervals. The acquisition unit 211 adds log information including one or more index values of the user who possesses the user terminal 2a to the log information DB 221 every time the predetermined period has passed in a time series. The acquisition unit 211 also adds the position information, the user information, and the time information included in the user data to the log information DB 221 as needed, as a step-by-step operation.

Similarly to the BD creation unit 312, the BD creation unit 212 creates the base data based on the log information stored in the log information DB 221 (step S22). In step S22, for example, the BD creation unit 212 calculates a statistical value such as an average value, a maximum value, or a minimum value for at least one index value included in the log information. The calculated statistical value configures the base data.

Similarly to the BD creation unit 312, the BD creation unit 212 may calculate the statistical value of the user under various conditions exemplified below. For example, the BD creation unit 212 calculates a statistical value such as an average value, a maximum value, or a minimum value for each time zone. For example, the BD creation unit 212 calculates a statistical value such as an average value, a maximum value, or a minimum value after smoking or before smoking. For example, the BD creation unit 212 calculates a statistical value such as an average value, a maximum value, or a minimum value of the amount of change before and after smoking. For example, the BD creation unit 212 calculates a statistical value such as an average value, a maximum value, or a minimum value of the user at a certain time point in a given day. For example, the BD creation unit 212 calculates a statistical value such as an average value, a maximum value, or a minimum value for each smoking area. For example, the BD creation unit 212 calculates a statistical value such as an average value, a maximum value, or a minimum value for each smoking area in each time zone. Not limited to the above, the BD creation unit 212 may calculate a statistical value by arbitrarily combining data included in the log information. The BD creation unit 212 creates base data including the above-described various statistical values related to the user who possesses the user terminal 2a. Furthermore, the user terminal 2a may acquire basic data of the user who possesses the user terminal 2a and create the base data by measuring the biometric data of the user at the time of initial use or registering the inhaling device 1a, or by measuring the biometric data of the user for a certain period such as a week.

According to this example, the user terminal 2a may compare one or more index values included in the latest log information with various thresholds of the user. The user terminal 2a can determine the stress state of the user in various perspectives. Further, the user terminal 2a may effectively determine the stress state of the user.

Similarly to the storage control unit 313, the storage control unit 213 performs control so that the base data created by the base data creation unit 212 is stored in the base data DB 222 (step S23). In step S23, for example, the storage control unit 213 accordingly adds the base data created by the base data creation unit 212 to the base data DB 222 every time the base data is created.

FIG. 22 is a flowchart showing an example of processing a recommendation by the user terminal according to the second embodiment of the present invention.

The processing procedure described below is merely an example, and each processing may be changed as much as possibility allows. In addition, in the processing procedure described below, steps can be omitted, replaced, and added as appropriate according to the embodiment.

In order to simplify the description, the user terminal 2a will be described below as an example.

The acquisition unit 211 acquires user data (step S31). For example, in step S31, the acquisition unit 211 acquires the user data from the storage unit 22. For example, the acquisition unit 211 acquires the biometric data measured in the inhaling device 1a from the storage unit 22, for example, in real time. For example, the acquisition unit 211 acquires position information associated with the biometric data from the storage unit 22. For example, the acquisition unit 211 acquires time information associated with the biometric data from the storage unit 22. For example, the acquisition unit 211 acquires user information from the storage unit 22. The acquisition unit 211 acquires at least one index value based on biometric data. For example, the acquisition unit 211 accordingly adds the log information including one or more index values to the log information DB 221 every time the predetermined period has passed in a time series. The acquisition unit 211 also accordingly adds the position information, the user information, and the time information to the log information DB 221 as needed.

Similarly to the comparison unit 314, the comparison unit 214 compares at least one index value acquired by the acquisition unit 211 with a threshold value (step S32). In step S32, for example, the comparison unit 214 compares the at least one index value acquired by the acquisition unit 211 and included in the latest log information with the threshold value. The comparison unit 214 may compare at least one index value included in the latest log information with the threshold value every time the latest log information is updated. The threshold value corresponds to an arbitrary statistical value included in the base data that is stored in the base data DB 222 and is related to the user who possesses the user terminal 2a. The threshold value may be a value higher than the arbitrary statistical value by a predetermined value. For example, the comparison unit 214 compares a "pulse rate" with a threshold value corresponding to a statistical value of an "average value of past logs" of pulse rates associated with the user ID of the user terminal 2a included in the base data.

The comparison unit 214 may compare, for each index, at least one index value with a threshold value corresponding to a statistical value associated with the user ID of the user terminal 2a. The comparison unit 214 may compare, for each index, at least one index value with a plurality of threshold values corresponding to a plurality of statistical values included in the base data. The comparison unit 214 may compare at least one index value with a threshold value corresponding to a statistical value under the same condition as that for acquiring the index value.

Similarly to the creation unit 315, the creation unit 215 creates stress state information based on at least one index value based on the comparison result of the comparison unit 214 (step S33). In step S33, for example, the creation unit 215 creates stress state information indicating a result of comparison between at least one index value and a threshold value as a stress state. According to this example, the user terminal 2a can provide the stress state of the user in an easily visible format such as text or a graph. The user terminal 2a can recommend the user to take a measure for relieving the stress.

As a result of comparing at least one index value with the threshold values in step S32, if the comparison unit 214 determines that the at least one index value is equal to or greater than at least one threshold value (step S34: YES), the process transitions from step S34 to step S35. As a result of comparing at least one index value with the threshold values in step S32, if the comparison unit 214 determines that no index value is equal to or greater than any threshold value (step S34: NO), the process transitions from step S34 to step S36.

Similarly to the selection unit 316, the selection unit 216 selects at least one recommendation in response to at least one index value being equal to or greater than the threshold value (step S35). In step S35, for example, the selection unit 216 selects at least one recommendation stored in the recommendation DB 224. The selection unit 216 selects at least one recommendation in consideration of the user information acquired by the acquisition unit 211. For example, the selection unit 216 selects a recommendation based on the stress state information as a recommendation included in the at least one recommendation. The selection unit 216 selects a recommendation based on information differing from the stress state information as a recommendation included in the at least one recommendation. The selection unit 216 selects a rest facility as a recommendation included in the at least one recommendation based on the position information. The recommendation and the selection mode of the recommendation are the same as those in the first embodiment. In this example, the user terminal 2a can recommend the user to take a measure for relieving the stress by the selected recommendation.

Similarly to the display control unit 317, the display control unit 217 controls at least display of the stress state information (step S36). In step S36, for example, in a case where the selection unit 216 does not select at least one recommendation, the display control unit 217 controls the display of the stress state information. For example, in a case where the selection unit 216 selects at least one recommendation, the display control unit 217 controls the display of the stress state information and the at least one recommendation.

The display control unit 217 causes the display unit 24 to display the stress state information created by the creation unit 215 in step S33 on the display unit 24. The display mode of the stress state information is the same as that in the first embodiment. According to this example, the user terminal 2a can provide the user stress state in an easily visible format. The user terminal 2a can recommend a measure for stress relief.

The display control unit 217 causes the display unit 24 to display one or more recommendations selected by the selection unit 216 in step S35. For example, the display control unit 217 controls the display of the recommendation based on the stress state information. For example, the display control unit 217 controls the display of the recommendation based on information differing from the stress state information. For example, the display control unit 217 controls the display of a map on which a rest facility is superimposed. The display mode of the recommendation is the same as that in the first embodiment. According to this example, the user terminal 2a can provide the recommendation in an easily visible format. The user terminal 2a can recommend a measure for stress relief.

In a case where the inhaling device 1a includes a display unit, similarly to the first embodiment, the display control unit 217 may control the display of the stress state information and at least one recommendation on the inhaling device 1a.

In step S34, if the comparison unit 214 determines that at least one index value is equal to or greater than at least one threshold value, the transmission unit 218 may transmit a control command. The transmission unit 218 transmits, to the inhaling device 1a, a control command for controlling the inhaling device 1a via the communication unit 25, in response to the at least one index value being equal to or greater than the threshold value. In this example, the communication unit 25 transmits the control command to the inhaling device 1a. According to this example, in a case where it is determined that the stress is high, the user terminal 2a can change the heating temperature, the smoke amount, and the like, of the inhaling device 1a. The user terminal 2a can control the inhaling state of the user and support escape from the stress state.

### <<Modification>>

The second embodiment may include the following modification in addition to the modification of the first embodiment.

In the second embodiment, an example has been described in which the BD creation unit 212 creates base data including various statistical values related to the user who possesses the user terminal 2a, but the embodiment is not limited to this. The acquisition unit 211 may acquire, from the information processing server 3 connected to the user terminal 2a, a statistical value of each group obtained by grouping a plurality of users according to an attribute and a statistical value of all users within the plurality of users. For example, the acquisition unit 211 acquires the statistical value from the information processing server 3 via the communication unit 25 at a predetermined cycle, such as once a month. The base data creation unit 212 creates base data based on the statistical values acquired by the acquisition unit 211.

According to this example, the user terminal 2a may create the stress state information using not only the statistical values related to the user who possesses the user terminal 2a but also the statistical values of the other users. Therefore, the user terminal 2 can more effectively determine the stress state. The user terminal 2a can recommend a measure for stress relief.

The statistical values acquired by the user terminal 2a from the information processing server 3 may be values from which certain information, such as private information, has been deleted. In addition, the user terminal 2a may acquire base data created in other user terminals 2 from the information processing server 3. The user terminal 2a may transmit the statistical values related to the user who possesses the user terminal 2a to the information processing server 3 at a predetermined cycle, such as once a month.

The present invention is not limited to the above-described embodiments, and can be modified in various manners in practice during implementation without departing from the gist of the invention. Moreover, the embodiments can be suitably combined; in that case, the combined advantages are obtained. Furthermore, the above-described embodiments include various inventions, and various inventions can be extracted by a combination selected from structural elements disclosed herein. For example, if the object of the invention is achieved and the advantages of the invention are attained even after some of the structural elements disclosed in connection with the embodiments are deleted, the structure made up of the resultant structural elements can be extracted as an invention.

### REFERENCE SIGNS LIST

1 ... inhaling device, 1a-1n ... inhaling device, 2 ... user terminal, 2a-2n ... user terminal, 3 ... information processing server, 10 ... information distribution system, 21 ... control unit, 22 ... storage unit, 23 ... input unit, 24 ... display unit, 25 ... communication unit, 26 ... detection unit, 31 ... control unit, 32 ... storage unit, 33 ... communication unit, 100A ... inhaling device, 100B ... inhaling device, 110 ... power-supply unit, 111A ... power supply, 111B ... power supply, 112A ... sensor unit, 112B ... sensor unit, 113A ... notification unit, 113B ... notification unit, 114A ... storage unit, 114B ... storage unit, 115A ... communication unit, 115B ... communication unit, 116A ... control unit, 116B ... control unit, 120 ... cartridge, 121A ... heating unit, 121B ... heating unit, 122 ... liquid guide unit, 123 ... liquid storage unit, 124 ... mouthpiece, 130 ... flavor-imparting cartridge, 131 ... flavor source, 140 ... holding part, 141 ... internal space, 142 ... opening, 143 ... bottom portion, 144 ... heat-insulating part, 150 ... stick-shaped base material, 151 ... base material portion, 152 ... suction portion, 180 ... airflow path, 181 ... air inflow hole, 182 ... air outflow hole, 190 ... arrow, 211 ... acquisition unit, 212 ... BD creation unit, 213 ... storage control unit, 214 ... comparison unit, 215 ... creation unit, 216 ... selection unit, 217 ... display control unit, 218 ... transmission unit, 221 ... log information DB, 222 ... base data DB, 223 ... stress state information DB, 224 ... recommendation DB, 225 ... map information DB, 311 ... acquisition unit, 312 ... BD creation unit, 313 ... storage control unit, 314 ... comparison unit, 315 ... creation unit, 316 ... selection unit, 317 ... display control unit, 318 ... transmission unit, 321 ... log information DB, 322 ... base data DB, 323 ... stress state information DB, 324 ... recommendation DB, 325 ... map information DB

## Claims

1. An information processing device comprising:
an acquisition unit configured to acquire at least one index value based on biometric data measured by a flavor inhaler;
a comparison unit configured to compare the at least one index value with a threshold value;
a creation unit configured to create, based on a comparison result of the comparison unit, stress state information based on the at least one index value;
a selection unit configured to select at least one recommendation according to the at least one index value; and
a display control unit configured to control display of the stress state information created by the creation unit and the at least one recommendation selected by the selection unit.

2. The information processing device according to claim 1, wherein
the selection unit is configured to select a recommendation based on the stress state information as a recommendation included in the at least one recommendation, and
the display control unit is configured to control display of the recommendation based on the stress state information selected by the selection unit.

3. The information processing device according to claim 1 or 2, wherein
the acquisition unit is configured to acquire position information on a user terminal connected to the flavor inhaler,
the selection unit is configured to select a rest facility as a recommendation included in the at least one recommendation based on the position information acquired by the acquisition unit, and
the display control unit is configured to control display of a map on which the rest facility selected by the selection unit is superimposed.

4. The information processing device according to any one of claims 1 to 3, wherein
the acquisition unit is configured to acquire user information, and
the selection unit is configured to select the at least one recommendation in consideration of the user information acquired by the acquisition unit.

5. The information processing device according to claim 2, wherein the recommendation based on the stress state information includes a message that recommends an action to reduce stress.

6. The information processing device according to any one of claims 1 to 5, further comprising a transmission unit configured to transmit a control command for controlling the flavor inhaler in accordance with the at least one index value.

7. An information processing method, comprising:
acquiring at least one index value based on biometric data measured by a flavor inhaler;
comparing the at least one index value with a threshold value;
creating, based on a result of the comparing, stress state information based on the at least one index value;
selecting at least one recommendation according to the at least one index value; and
controlling display of the created stress state information and the selected at least one recommendation.

8. The information processing method according to claim 7, wherein
the selecting includes selecting a recommendation based on the stress state information as a recommendation included in the at least one recommendation, and
the controlling display includes controlling display of the selected recommendation based on the stress state information.

9. The information processing method according to claim 7 or 8, wherein
the acquiring includes acquiring position information on a user terminal connected to the flavor inhaler,
the selecting includes selecting a rest facility as a recommendation included in the at least one recommendation based on the acquired position information, and
the controlling display includes controlling display of a map on which the selected rest facility is superimposed.

10. The information processing method according to any one of claims 7 to 9, wherein
the acquiring includes acquiring user information, and
the selecting includes selecting the at least one recommendation in consideration of the acquired user information.

11. The information processing method according to claim 8, wherein the recommendation based on the stress state information includes a message that recommends an action to reduce stress.

12. The information processing method according to any one of claims 7 to 11, further comprising transmitting a control command for controlling the flavor inhaler in accordance with the at least one index value.

13. A program that causes a computer to realize:
an acquisition unit configured to acquire at least one index value based on biometric data measured by a flavor inhaler;
a comparison unit configured to compare the at least one index value with a threshold value;
a creation unit configured to create, based on a comparison result of the comparison unit, stress state information based on the at least one index value;
a selection unit configured to select at least one recommendation according to the at least one index value; and
a display control unit configured to control display of the stress state information created by the creation unit and the at least one recommendation selected by the selection unit.
